# EUROPEAN PATENT APPLICATION

(11) **EP 4 473 970 A1**
(43) Date of publication of application: **11.12.2024**
(21) Application number: 23780756.5
(22) Date of filing: 29.03.2023
(51) Int. Cl.: A61K 31/53, A61K 31/519, A61K 45/00, A61P 35/00, A61P 43/00

(54) **PHARMACEUTICAL COMPOSITION FOR TREATING TUMORS**

(30) Priority: 31.03.2022 JP 2022060715
(71) Applicant: Eisai R&D Management Co., Ltd., Tokyo 112-8088 (JP)
(72) Inventor: KATO Yu, Tsukuba-shi, Ibaraki 300-2635 (JP); KUME Masahiko, Tsukuba-shi, Ibaraki 300-2635 (JP); HORI Yusaku, Tsukuba-shi, Ibaraki 300-2635 (JP); ADACHI Yusuke, Tsukuba-shi, Ibaraki 300-2635 (JP)
(74) Representative: Hoffmann Eitle
(86) International application number: PCT/JP2023/012957
(87) International publication number: WO 2023/190748

(57) **Abstract**

The present invention provides a pharmaceutical composition for treating a tumor, the pharmaceutical composition comprising (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydr o-2H-pyrazino[2,1-c][1,2,4]triazine-1(6H)-carboxamide represented by formula (I) or a pharmaceutically acceptable salt thereof, wherein the pharmaceutical composition is administered in combination with an RAS inhibitor.

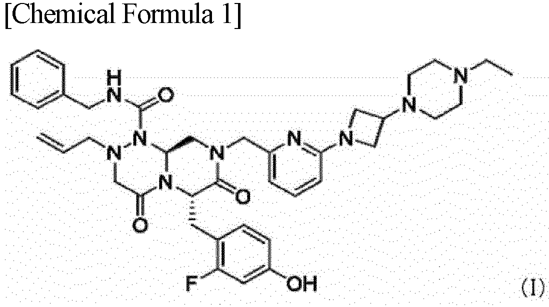

## Description

### Technical Field

The present invention relates to a pharmaceutical composition for treating a tumor.

### Background Art

Ras proteins belong to a family of related proteins that are present in all eukaryotic organisms from yeast to human (Non Patent Literature 1). The occurrence of amino acid substitution owing to mutation of an RAS gene is believed to cause the reduction of GTPase activity of Ras protein and the decrease of affinity with GAP, resulting in the increase in the proportion of active forms of Ras protein. Excessive signaling enhancement due to this is considered to lead to oncogenesis and the growth and proliferation of cancer. There are four major Ras proteins [KRAS (KRAS4A and KRAS4B), NRAS, HRAS] that are generated from three genes: a KRAS gene, an NRAS gene, and an BRAS gene. KRAS is the most frequently mutated member in the RAS family, and believed to be the most common oncogenic gene driver for cancers in human. Mutation of KRAS is generally observed in non-small cell lung cancer, colorectal cancer, and pancreatic cancer (Non Patent Literature 2).

A Wnt/β catenin signaling pathway, a signaling pathway highly conserved in the course of the evolution of animals, regulates gene expressions involved in the growth and differentiation of cells, axis formation, organogenesis, and so on. It is known that an abnormal activation of the Wnt/β catenin signaling pathway is observed in various cancers such as colorectal cancer and hepatocellular cancer (Non Patent Literature 3).

The IUPAC name of a compound represented by formula (I) is (6S,9aS)-N-benzyl-8-({6-[3-(4-ethylpiperazin-1-yl)azetidin-1-yl]pyridin-2-yl} methyl)-6-(2-fhioro-4-hydroxybenzyl)-4,7-dioxo-2-(prop-2-en-1-yl)hexahydr o-2H-pyrazino[2,1-c][1,2,4]triazine-1(6H)-carboxamide, and the compound represented by formula (I) is also referred to as (6S,9aS)-N-benzyl-8-((6-(3-(4-ethylpiperazin-1-yl)azetidin-1-yl)pyridin-2-yl) methyl)-6-((2-fluoro-4-hydroxyphenyl)methyl)-4,7-dioxo-2-(prop-2-en-1-yl)-octahydro-1H-pyrazino[2,1-c][1,2,4]triazine-1-carboxamide and known as a compound that exhibits Wnt pathway-modulating activity (Patent Literatures 1, 2).

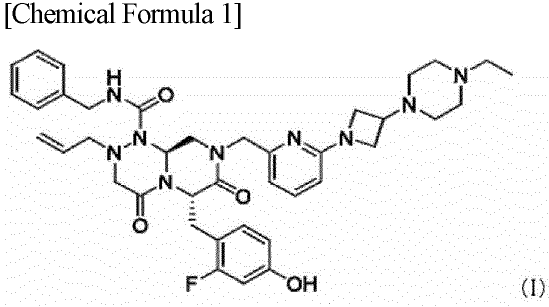

### Citation List

### Patent Literature

Patent Literature 1: WO 2015/098853
Patent Literature 2: WO 2016/208576

### Non Patent Literature

Non Patent Literature 1: Santos et al., Structural and functional properties of ras proteins, FASEB J. 1989, 3(10), 2151-2163
Non Patent Literature 2: Interaction between Wnt/β-catenin and RAS-ERK pathways and an anti-cancer strategy via degradations of β-catenin and RAS by targeting the Wnt/β-catenin pathway, npj Precision One 2, 5 (2018)
Non Patent Literature 3: Fodde, et al., Wnt/β-catenin signaling in cancer stemness and malignant behavior, Current Opinion in Cell Biology 2007, 19:150-158

### Summary of Invention

### Technical Problem

Development of drugs targeting RAS has been actively attempted in recent years. Regarding molecular targeting drugs for KRAS mutation, for example, the KRAS G12C inhibitor, sotorasib is clinically used as a therapeutic agent for non-small cell lung cancer with KRAS G12 mutation. However, combination therapy of an RAS inhibitor and a Wnt/β-catenin pathway inhibitor is still unknown.

An object of the present invention is to provide a novel pharmaceutical composition for treating a tumor.

### Solution to Problem

The present inventors have diligently examined to find that combined administration of (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydr o-2H-pyrazino[2,1-c][1,2,4]triazine-1(6H)-carboxamide (hereinafter, also referred to as the compound represented by formula (I)) or a pharmaceutically acceptable salt thereof and an RAS inhibitor exhibits high antitumor effect, and thus completed the present invention.

Specifically, the present disclosure is as follows.
[1] A pharmaceutical composition for treating a tumor, the pharmaceutical composition comprising
   (6S,9aS)-N-benzyl-8-({6-[3-(4-ethylpiperazin-1-yl)azetidin-1-yl]pyri din-2-yl}methyl)-6-(2-fluoro-4-hydroxybenzyl)-4,7-dioxo-2-(prop-2-en-1-yl) hexahydro-2H-pyrazino[2,1-c][1,2,4]triazine-1(6H)-carboxamide represented by formula (I) or a pharmaceutically acceptable salt thereof, wherein
   the pharmaceutical composition is administered in combination with an RAS inhibitor 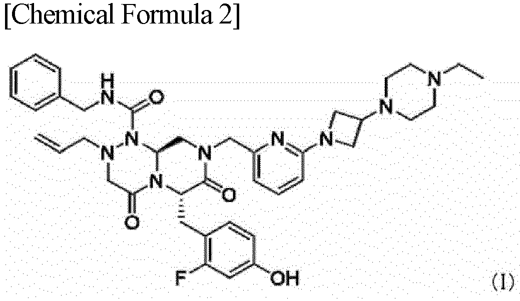
[2] The pharmaceutical composition according to [1], wherein the (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydr o-2H pyrazino[2,1-c][1,2,4]triazine-1(6H)-carboxamide or pharmaceutically acceptable salt thereof is administered with the RAS inhibitor simultaneously or at different times.
[3] The pharmaceutical composition according to [1] or [2], wherein the (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydr o-2H-pyrazino[2,1-c][1,2,4]triazine-1(6H)-carboxamide or pharmaceutically acceptable salt thereof is (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydr o-2H-pyrazino[2,1-c][1,2,4]triazine-1(6H)-carboxamide.
[4] The pharmaceutical composition according to any one of [1] to [3], wherein the RAS inhibitor is a KRAS inhibitor.
[5] The pharmaceutical composition according to [4], wherein the KRAS inhibitor is at least one selected from sotorasib (AMG 510), adagrasib (MRTX849), MRTX1133, BI-3406, BI 1701963, RG6330, LY3537982, JDQ443, RMC-6291, RMC-6236, LUNA18, ARS-1620, JNJ-74699157, GDC-6036, iExosomes, mRNA-5671, JAB-21822, BI 1823911, MK-1084, ELI-002, SDGR5, and JAB-22000.
[6] The pharmaceutical composition according to [4], wherein the KRAS inhibitor is sotorasib, adagrasib, or BI 1701963.
[7] The pharmaceutical composition according to [4], wherein the KRAS inhibitor is a KRAS G12C inhibitor.
[8] The pharmaceutical composition according to [7], wherein the KRAS G12C inhibitor is sotorasib or adagrasib.
[9] The pharmaceutical composition according to any one of [1] to [8], wherein the tumor is at least one selected from non-small cell lung cancer, colorectal cancer, pancreatic cancer, mesothelioma, endometrial cancer, ovarian cancer, bladder cancer, bile duct cancer, gastric cancer, breast cancer, small cell lung cancer, testis cancer, small intestine cancer, appendix cancer, and neuroendocrine tumor.
[10] The pharmaceutical composition according to any one of [1] to [8], wherein the tumor is at least one selected from non-small cell lung cancer, colorectal cancer, pancreatic cancer, endometrial cancer, and ovarian cancer.
[11] The pharmaceutical composition according to any one of [1] to [8], wherein the tumor is at least one selected from non-small cell lung cancer and colorectal cancer.
[12] A pharmaceutical composition for treating a tumor, the pharmaceutical composition comprising an RAS inhibitor, wherein
   the pharmaceutical composition is administered in combination with (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydr o-2H-pyrazino[2,1-c][1,2,4]triazine-1(6H)-carboxamide represented by formula (I) or a pharmaceutically acceptable salt thereof 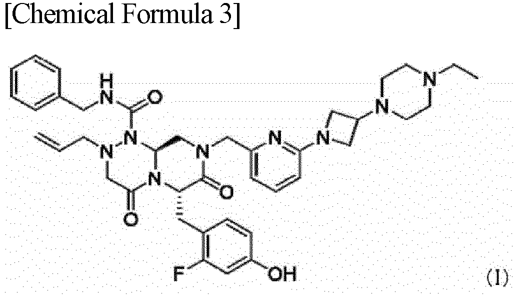
[13] The pharmaceutical composition according to [12], wherein the RAS inhibitor is administered with the (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydr o-2H-pyrazino[2,1-c][1,2,4]triazine-1(6H)-carboxamide or pharmaceutically acceptable salt thereof simultaneously or at different times.
[14] The pharmaceutical composition according to [12] or [13], wherein the (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydr o-2H-pyrazino[2,1-c][1,2,4]triazine-1(6H)-carboxamide or pharmaceutically acceptable salt thereof is (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydr o-2H-pyrazino[2,1-c][1,2,4]triazine-1(6H)-carboxamide.
[15] The pharmaceutical composition according to any one of [12] to [14], wherein the RAS inhibitor is a KRAS inhibitor.
[16] The pharmaceutical composition according to [15], wherein the KRAS inhibitor is at least one selected from sotorasib, adagrasib, MRTX1133, BI-3406, BI 1701963, RG6330, LY3537982, JDQ443, RMC-6291, RMC-6236, LUNA18, ARS-1620, JNJ-74699157, GDC-6036, iExosomes, mRNA-5671, JAB-21822, BI 1823911, MK-1084, ELI-002, SDGR5, and JAB-22000.
[17] The pharmaceutical composition according to [15], wherein the KRAS inhibitor is sotorasib, adagrasib, or BI 1701963.
[18] The pharmaceutical composition according to [15], wherein the KRAS inhibitor is a KRAS G12C inhibitor.
[19] The pharmaceutical composition according to [18], wherein the KRAS G12C inhibitor is sotorasib or adagrasib.
[20] The pharmaceutical composition according to any one of [12] to [19], wherein the tumor is at least one selected from non-small cell lung cancer, colorectal cancer, pancreatic cancer, mesothelioma, endometrial cancer, ovarian cancer, bladder cancer, bile duct cancer, gastric cancer, breast cancer, small cell lung cancer, testis cancer, small intestine cancer, appendix cancer, and neuroendocrine tumor.
[21] The pharmaceutical composition according to any one of [12] to [19], wherein the tumor is at least one selected from non-small cell lung cancer, colorectal cancer, pancreatic cancer, endometrial cancer, and ovarian cancer.
[22] The pharmaceutical composition according to any one of [12] to [19], wherein the tumor is at least one selected from non-small cell lung cancer and colorectal cancer.
[23] A therapeutic agent for a tumor, the therapeutic agent comprising (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydr o-2H-pyrazino[2,1-c][1,2,4]triazine-1(6H)-carboxamide represented by formula (I) or a pharmaceutically acceptable salt thereof, wherein
   the therapeutic agent is administered in combination with an RAS inhibitor 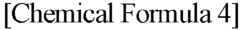 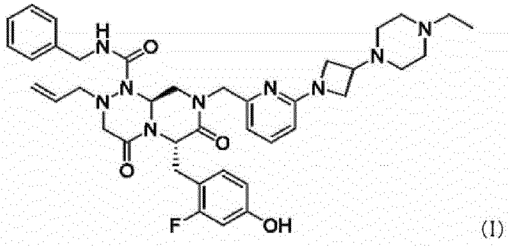
[24] The therapeutic agent according to [23], wherein the (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydr o-2H pyrazino[2,1-c][1,2,4]triazine-1(6H)-carboxamide or pharmaceutically acceptable salt thereof is administered with the RAS inhibitor simultaneously or at different times.
[25] The therapeutic agent according to [23] or [24], wherein the (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydr o-2H-pyrazino[2,1-c][1,2,4]triazine-1(6H)-carboxamide or pharmaceutically acceptable salt thereof is (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydr o-2H-pyrazino[2,1-c][1,2,4]triazine-1(6H)-carboxamide.
[26] The therapeutic agent according to any one of [23] to [25], wherein the RAS inhibitor is a KRAS inhibitor.
[27] The therapeutic agent according to [26], wherein the KRAS inhibitor is at least one selected from sotorasib, adagrasib, MRTX1133, BI-3406, BI 1701963, RG6330, LY3537982, JDQ443, RMC-6291, RMC-6236, LUNA18, ARS-1620, JNJ-74699157, GDC-6036, iExosomes, mRNA-5671, JAB-21822, BI 1823911, MK-1084, ELI-002, SDGR5, and JAB-22000.
[28] The therapeutic agent according to [26], wherein the KRAS inhibitor is sotorasib, adagrasib, or BI 1701963.
[29] The therapeutic agent according to [26], wherein the KRAS inhibitor is a KRAS G12C inhibitor.
[30] The therapeutic agent according to [29], wherein the KRAS G12C inhibitor is sotorasib or adagrasib.
[31] The therapeutic agent according to any one of [23] to [30], wherein the tumor is at least one selected from non-small cell lung cancer, colorectal cancer, pancreatic cancer, mesothelioma, endometrial cancer, ovarian cancer, bladder cancer, bile duct cancer, gastric cancer, breast cancer, small cell lung cancer, testis cancer, small intestine cancer, appendix cancer, and neuroendocrine tumor.
[32] The therapeutic agent according to any one of [23] to [30], wherein the tumor is at least one selected from non-small cell lung cancer, colorectal cancer, pancreatic cancer, endometrial cancer, and ovarian cancer.
[33] The therapeutic agent according to any one of [23] to [30], wherein the tumor is at least one selected from non-small cell lung cancer and colorectal cancer.
[34] A therapeutic agent for a tumor, the therapeutic agent comprising an RAS inhibitor, wherein
   the therapeutic agent is administered in combination with (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydr o-2H-pyrazino[2,1-c][1,2,4]triazine-1(6H)-carboxamide represented by formula (I)
   or a pharmaceutically acceptable salt thereof 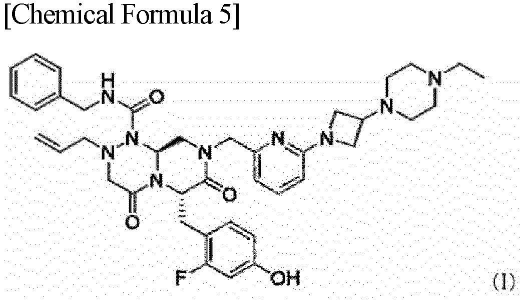
[35] The therapeutic agent according to [34], wherein the RAS inhibitor is administered with the (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydr o-2H pyrazino[2,1-c][1,2,4]triazine-1(6H)-carboxamide or pharmaceutically acceptable salt thereof simultaneously or at different times.
[36] The therapeutic agent according to [34] or [35], wherein the (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydr o-2H pyrazino[2,1-c][1,2,4]triazine-1(6H)-carboxamide or pharmaceutically acceptable salt thereof is (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydr o-2H-pyrazino[2,1-c][1,2,4]triazine-1(6H)-carboxamide.
[37] The therapeutic agent according to any one of [34] to [36], wherein the RAS inhibitor is a KRAS inhibitor.
[38] The therapeutic agent according to [37], wherein the KRAS inhibitor is at least one selected from sotorasib, adagrasib, MRTX1133, BI-3406, BI 1701963, RG6330, LY3537982, JDQ443, RMC-6291, RMC-6236, LUNA18, ARS-1620, JNJ-74699157, GDC-6036, iExosomes, mRNA-5671, JAB-21822, BI 1823911, MK-1084, ELI-002, SDGR5, and JAB-22000.
[39] The therapeutic agent according to [37], wherein the KRAS inhibitor is sotorasib, adagrasib, or BI 1701963.
[40] The therapeutic agent according to [37], wherein the KRAS inhibitor is a KRAS G12C inhibitor.
[41] The therapeutic agent according to [40], wherein the KRAS G12C inhibitor is sotorasib or adagrasib.
[42] The therapeutic agent according to any one of [34] to [41], wherein the tumor is at least one selected from non-small cell lung cancer, colorectal cancer, pancreatic cancer, mesothelioma, endometrial cancer, ovarian cancer, bladder cancer, bile duct cancer, gastric cancer, breast cancer, small cell lung cancer, testis cancer, small intestine cancer, appendix cancer, and neuroendocrine tumor.
[43] The therapeutic agent according to any one of [34] to [41], wherein the tumor is at least one selected from non-small cell lung cancer, colorectal cancer, pancreatic cancer, endometrial cancer, and ovarian cancer.
[44] The therapeutic agent according to any one of [34] to [41], wherein the tumor is at least one selected from non-small cell lung cancer and colorectal cancer.
[45] A method of treating a tumor, comprising administering to a patient in need thereof (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydr o-2H-pyrazino[2,1-c][1,2,4]triazine-1(6H)-carboxamide represented by formula (I) or a pharmaceutically acceptable salt thereof and an RAS inhibitor 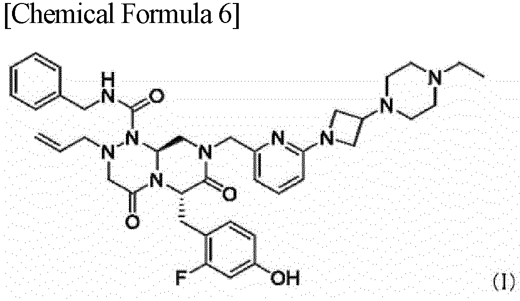
[46] The method according to [45], wherein the (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydr o-2H-pyrazino[2,1-c][1,2,4]triazine-1(6H)-carboxamide or pharmaceutically acceptable salt thereof and the RAS inhibitor are administered with each other simultaneously or at different times.
[47] The method according to [45] or [46], wherein the (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydr o-2H-pyrazino[2,1-c][1,2,4]triazine-1(6H)-carboxamide or pharmaceutically acceptable salt thereof is (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydr o-2H-pyrazino[2,1-c][1,2,4]triazine-1(6H)-carboxamide.
[48] The method according to any one of [45] to [47], wherein the RAS inhibitor is a KRAS inhibitor.
[49] The method according to [48], wherein the KRAS inhibitor is at least one selected from sotorasib (AMG 510), adagrasib (MRTX849), MRTX1133, BI-3406, BI 1701963, RG6330, LY3537982, JDQ443, RMC-6291, RMC-6236, LUNA18, ARS-1620, JNJ-74699157, GDC-6036, iExosomes, mRNA-5671, JAB-21822, BI 1823911, MK-1084, ELI-002, SDGR5, and JAB-22000.
[50] The method according to [48], wherein the KRAS inhibitor is sotorasib, adagrasib, or BI 1701963.
[51] The method according to [48], wherein the KRAS inhibitor is a KRAS G12C inhibitor.
[52] The method according to [51], wherein the KRAS G12C inhibitor is sotorasib or adagrasib.
[53] The method according to any one of [45] to [52], wherein the tumor is at least one selected from non-small cell lung cancer, colorectal cancer, pancreatic cancer, mesothelioma, endometrial cancer, ovarian cancer, bladder cancer, bile duct cancer, gastric cancer, breast cancer, small cell lung cancer, testis cancer, small intestine cancer, appendix cancer, and neuroendocrine tumor.
[54] The method according to any one of [45] to [52], wherein the tumor is at least one selected from non-small cell lung cancer, colorectal cancer, pancreatic cancer, endometrial cancer, and ovarian cancer.
[55] The method according to any one of [45] to [52], wherein the tumor is at least one selected from non-small cell lung cancer and colorectal cancer.
[56] Use of (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydr o-2H-pyrazino[2,1-c][1,2,4]triazine-1(6H)-carboxamide represented by formula (I) or a pharmaceutically acceptable salt thereof, in the manufacture of a pharmaceutical composition for the treatment of a tumor, wherein the pharmaceutical composition is administered in combination with an RAS inhibitor 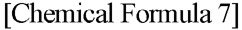 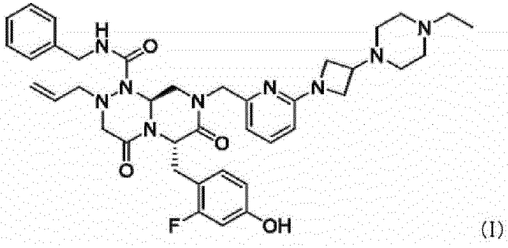
[57] The use according to [56], wherein the pharmaceutical composition for the treatment of a tumor comprising (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydr o-2H pyrazino[2,1-c][1,2,4]triazine-1(6H)-carboxamide or pharmaceutically acceptable salt thereof is administered with the RAS inhibitor simultaneously or at different times.
[58] The use according to [56] or [57], wherein the (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydr o-2H-pyrazino[2,1-c][1,2,4]triazine-1(6H)-carboxamide or pharmaceutically acceptable salt thereof is (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydr o-2H-pyrazino[2,1-c][1,2,4]triazine-1(6H)-carboxamide.
[59] The use according to any one of [56] to [58], wherein the RAS inhibitor is a KRAS inhibitor.
[60] The use according to [59], wherein the KRAS inhibitor is at least one selected from sotorasib, adagrasib, MRTX1133, BI-3406, BI 1701963, RG6330, LY3537982, JDQ443, RMC-6291, RMC-6236, LUNA18, ARS-1620, JNJ-74699157, GDC-6036, iExosomes, mRNA-5671, JAB-21822, BI 1823911, MK-1084, ELI-002, SDGR5, and JAB-22000.
[61] The use according to [59], wherein the KRAS inhibitor is sotorasib, adagrasib, or BI 1701963.
[62] The use according to [59], wherein the KRAS inhibitor is a KRAS G12C inhibitor.
[63] The use according to [62], wherein the KRAS G12C inhibitor is sotorasib or adagrasib.
[64] The use according to any one of [56] to [63], wherein the tumor is at least one selected from non-small cell lung cancer, colorectal cancer, pancreatic cancer, mesothelioma, endometrial cancer, ovarian cancer, bladder cancer, bile duct cancer, gastric cancer, breast cancer, small cell lung cancer, testis cancer, small intestine cancer, appendix cancer, and neuroendocrine tumor.
[65] The use according to any one of [56] to [63], wherein the tumor is at least one selected from non-small cell lung cancer, colorectal cancer, pancreatic cancer, endometrial cancer, and ovarian cancer.
[66] The use according to any one of [56] to [63], wherein the tumor is at least one selected from non-small cell lung cancer and colorectal cancer.
[67] Use of an RAS inhibitor, in the manufacture of a pharmaceutical composition for the treatment of a tumor, wherein the pharmaceutical composition is administered in combination with (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydr o-2H-pyrazino[2,1-c][1,2,4]triazine-1(6H)-carboxamide represented by formula (I) or a pharmaceutically acceptable salt thereof 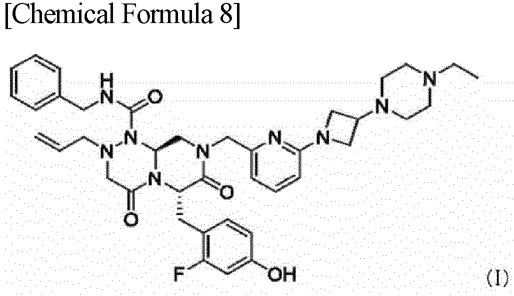
[68] The use according to [67], wherein the RAS inhibitor is administered with the (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydr o-2H-pyrazino[2,1-c][1,2,4]triazine-1(6H)-carboxamide or pharmaceutically acceptable salt thereof simultaneously or at different times.
[69] The use according to [67] or [68], wherein the (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydr o-2H pyrazino[2,1-c][1,2,4]triazine-1(6H)-carboxamide or pharmaceutically acceptable salt thereof is (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydr o-2H-pyrazino[2,1-c][1,2,4]triazine-1(6H)-carboxamide.
[70] The use according to any one of [67] to [69], wherein the RAS inhibitor is a KRAS inhibitor.
[71] The use according to [70], wherein the KRAS inhibitor is at least one selected from sotorasib, adagrasib, MRTX1133, BI-3406, BI 1701963, RG6330, LY3537982, JDQ443, RMC-6291, RMC-6236, LUNA18, ARS-1620, JNJ-74699157, GDC-6036, iExosomes, mRNA-5671, JAB-21822, BI 1823911, MK-1084, ELI-002, SDGR5, and JAB-22000.
[72] The use according to [70], wherein the KRAS inhibitor is sotorasib, adagrasib, or BI 1701963.
[73] The use according to [70], wherein the KRAS inhibitor is a KRAS G12C inhibitor.
[74] The use according to [73], wherein the KRAS G12C inhibitor is sotorasib or adagrasib.
[75] The use according to any one of [67] to [74], wherein the tumor is at least one selected from non-small cell lung cancer, colorectal cancer, pancreatic cancer, mesothelioma, endometrial cancer, ovarian cancer, bladder cancer, bile duct cancer, gastric cancer, breast cancer, small cell lung cancer, testis cancer, small intestine cancer, appendix cancer, and neuroendocrine tumor.
[76] The use according to any one of [67] to [74], wherein the tumor is at least one selected from non-small cell lung cancer, colorectal cancer, pancreatic cancer, endometrial cancer, and ovarian cancer.
[77] The use according to any one of [67] to [74], wherein the tumor is at least one selected from non-small cell lung cancer and colorectal cancer.
[78] (6S,9aS)-N-Benzyl-8-({6-[3-(4-ethylpiperazin-1-yl)azetidin-1-yl]pyridin-2-yl }methyl)-6-(2-fluoro-4-hydroxybenzyl)-4,7-dioxo-2-(prop-2-en-1-yl)hexahyd ro- 2H -pyrazino[2,-c] [1,2,4]triazine-1(6H)-carboxamide represented by formula (I) or a pharmaceutically acceptable salt thereof, for use in the treatment of a tumor, wherein the (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydr o-2H-pyrazino[2,1-c][1,2,4]triazine-1(6H)-carboxamide or pharmaceutically acceptable salt thereof is administered in combination with an RAS inhibitor 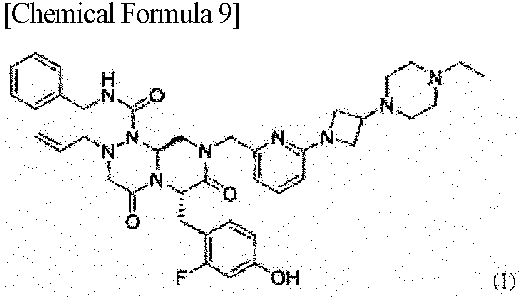
[79] The (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydr o-2H-pyrazino[2,1-c][1,2,4]triazine-1(6H)-carboxamide or pharmaceutically acceptable salt thereof for use according to [78], wherein the (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydr o-2H pyrazino[2,1-c][1,2,4]triazine-1(6H)-carboxamide or pharmaceutically acceptable salt thereof is administered with the RAS inhibitor simultaneously or at different times.
[80] The (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydr o-2H-pyrazino[2,1-c][1,2,4]triazine-1(6H)-carboxamide or pharmaceutically acceptable salt thereof for use according to [78] or [79], wherein the (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydr o-2H pyrazino[2,1-c][1,2,4]triazine-1(6H)-carboxamide or pharmaceutically acceptable salt thereof is (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1 -yl)hexahydr o-2H-pyrazino[2,1-c][1,2,4]triazine-1(6H)-carboxamide.
[81] The (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydr o-2H pyrazino[2,1-c][1,2,4]triazine-1(6H)-carboxamide or pharmaceutically acceptable salt thereof for use according to any one of [78] to [80], wherein the RAS inhibitor is a KRAS inhibitor.
[82] The (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydr o-2H pyrazino[2,1-c][1,2,4]triazine-1(6H)-carboxamide or pharmaceutically acceptable salt thereof for use according to [81], wherein the KRAS inhibitor is at least one selected from sotorasib, adagrasib, MRTX1133, BI-3406, BI 1701963, RG6330, LY3537982, JDQ443, RMC-6291, RMC-6236, LUNA18, ARS-1620, JNJ-74699157, GDC-6036, iExosomes, mRNA-5671, JAB-21822, BI 1823911, MK-1084, ELI-002, SDGR5, and JAB-22000.
[83] The (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydr o-2H-pyrazino[2,1-c][1,2,4]triazine-1(6H)-carboxamide or pharmaceutically acceptable salt thereof for use according to [81], wherein the KRAS inhibitor is sotorasib, adagrasib, or BI 1701963.
[84] The (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydr o-2H pyrazino[2,1-c][1,2,4]triazine-1(6H)-carboxamide or pharmaceutically acceptable salt thereof for use according to [81], wherein the KRAS inhibitor is a KRAS G12C inhibitor.
[85] The (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydr o-2H pyrazino[2,1-c][1,2,4]triazine-1(6H)-carboxamide or pharmaceutically acceptable salt thereof for use according to [84], wherein the KRAS G12C inhibitor is sotorasib or adagrasib.
[86] The (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydr o-2H pyrazino[2,1-c][1,2,4]triazine-1(6H)-carboxamide or pharmaceutically acceptable salt thereof for use according to any one of [78] to [85], wherein the tumor is at least one selected from non-small cell lung cancer, colorectal cancer, pancreatic cancer, mesothelioma, endometrial cancer, ovarian cancer, bladder cancer, bile duct cancer, gastric cancer, breast cancer, small cell lung cancer, testis cancer, small intestine cancer, appendix cancer, and neuroendocrine tumor.
[87] The (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydr o-2H-pyrazino[2,1-c][1,2,4]triazine-1(6H)-carboxamide or pharmaceutically acceptable salt thereof for use according to any one of [78] to [85], wherein the tumor is at least one selected from non-small cell lung cancer, colorectal cancer, pancreatic cancer, endometrial cancer, and ovarian cancer.
[88] The (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydr o-2H pyrazino[2,1-c][1,2,4]triazine-1(6H)-carboxamide or pharmaceutically acceptable salt thereof for use according to any one of [78] to [85], wherein the tumor is at least one selected from non-small cell lung cancer and colorectal cancer.
[89] An RAS inhibitor for use in treating a tumor, wherein the RAS inhibitor is administered in combination with (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydr o-2H-pyrazino[2,1-c][1,2,4]triazine-1(6H)-carboxamide represented by formula (I) or a pharmaceutically acceptable salt thereof 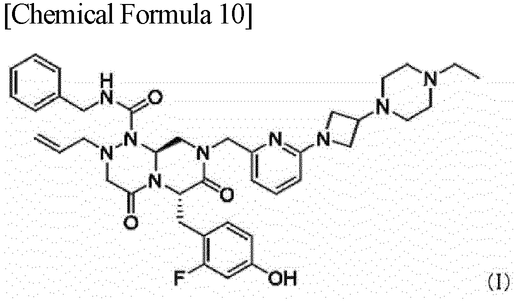
[90] The RAS inhibitor according to [89], wherein the RAS inhibitor is administered with the (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydr o-2H pyrazino[2,1-c][1,2,4]triazine-1(6H)-carboxamide or pharmaceutically acceptable salt thereof simultaneously or at different times.
[91] The RAS inhibitor according to [89] or [90], wherein the (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydr o-2H-pyrazino[2,1-c][1,2,4]triazine-1(6H)-carboxamide or pharmaceutically acceptable salt thereof is (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydr o-2H-pyrazino[2,1-c][1,2,4]triazine-1(6H)-carboxamide.
[92] The RAS inhibitor according to any one of [89] to [91], wherein the RAS inhibitor is a KRAS inhibitor.
[93] The RAS inhibitor according to [92], wherein the KRAS inhibitor is at least one selected from sotorasib, adagrasib, MRTX1133, BI-3406, BI 1701963, RG6330, LY3537982, JDQ443, RMC-6291, RMC-6236, LUNA18, ARS-1620, JNJ-74699157, GDC-6036, iExosomes, mRNA-5671, JAB-21822, BI 1823911, MK-1084, ELI-002, SDGR5, and JAB-22000.
[94] The RAS inhibitor according to [92], wherein the KRAS inhibitor is sotorasib, adagrasib, or BI 1701963.
[95] The RAS inhibitor according to [92], wherein the KRAS inhibitor is a KRAS G12C inhibitor.
[96] The RAS inhibitor according to [95], wherein the KRAS G12C inhibitor is sotorasib or adagrasib.
[97] The RAS inhibitor according to any one of [89] to [96], wherein the tumor is at least one selected from non-small cell lung cancer, colorectal cancer, pancreatic cancer, mesothelioma, endometrial cancer, ovarian cancer, bladder cancer, bile duct cancer, gastric cancer, breast cancer, small cell lung cancer, testis cancer, small intestine cancer, appendix cancer, and neuroendocrine tumor.
[98] The RAS inhibitor according to any one of [89] to [96], wherein the tumor is at least one selected from non-small cell lung cancer, colorectal cancer, pancreatic cancer, endometrial cancer, and ovarian cancer.
[99] The RAS inhibitor according to any one of [89] to [96], wherein the tumor is at least one selected from non-small cell lung cancer and colorectal cancer.
[100] The pharmaceutical composition, therapeutic agent, or use according to any one of [1] to [11], [23] to [33], and [56] to [66], wherein the pharmaceutical composition or therapeutic agent comprising (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydr o-2H pyrazino[2,1-c][1,2,4]triazine-1(6H)-carboxamide or a pharmaceutically acceptable salt thereof is for oral administration.
[101] The pharmaceutical composition, therapeutic agent, method, use, compound or pharmaceutically acceptable salt thereof for use, or RAS inhibitor for use according to any one of [12] to [22], [34] to [55], and [67] to [99], wherein the (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydr o-2H pyrazino[2,1-c][1,2,4]triazine-1(6H)-carboxamide or pharmaceutically acceptable salt thereof is orally administered.
[102] The pharmaceutical composition, therapeutic agent, method, use, compound or pharmaceutically acceptable salt thereof for use, or RAS inhibitor for use according to any one of [1] to [8], [12] to [19], [23] to [30], [34] to [41], [45] to [52], [56] to [63], [67] to [74], [78] to [85], and [89] to [96], wherein the tumor is at least one selected from non-small cell lung cancer, colorectal cancer, pancreatic cancer, endometrial cancer, bile duct cancer, and bladder cancer.

### Advantageous Effects of Invention

Combined administration of (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydr o-2H-pyrazino[2,1-c][1,2,4]triazine-1(6H)-carboxamide or a pharmaceutically acceptable salt thereof and an RAS inhibitor exhibits high antitumor effect.

### Brief Description of Drawings

[Figure 1] Figure 1 is a graph showing antitumor effects of combined administration of the compound represented by formula (I) (abbreviated as E7386 in the figure) and a KRAS inhibitor in the KRAS G12C mutation-positive human non-small cell lung cancer cell line NCI-H358.
[Figure 2] Figure 2 is a graph showing antitumor effects of combined administration of the compound represented by formula (I) (abbreviated as E7386 in the figure) and a KRAS inhibitor in a xenograft model with the KRAS G12C mutation-positive human non-small cell lung cancer cell line NCI-H358.
[Figure 3A] Figure 3Ais a graph showing cell growth inhibitory activity given by combined treatment with the compound represented by formula (I) (abbreviated as E7386 in the figure) and a KRAS inhibitor in the KRAS G12C mutation-positive human pancreatic cancer cell line MIA PaCa-2.
[Figure 3B] Figure 3B is a graph showing cell growth inhibitory activity given by combined treatment with the compound represented by formula (I) (abbreviated as E7386 in the figure) and a KRAS inhibitor in the KRAS G12C mutation-positive human bladder cancer cell line UM-UC-3.
[Figure 3C] Figure 3C is a graph showing cell growth inhibitory activity given by combined treatment with the compound represented by formula (I) (abbreviated as E7386 in the figure) and a KRAS inhibitor in the KRAS G12C mutation-positive human non-small cell lung cancer cell line NCI-H358.
[Figure 3D] Figure 3D is a graph showing cell growth inhibitory activity given by combined treatment with the compound represented by formula (I) (abbreviated as E7386 in the figure) and a KRAS inhibitor in the KRAS G12D mutation-positive human bile duct cancer cell line SNU-869.
[Figure 3E] Figure 3E is a graph showing cell growth inhibitory activity given by combined treatment with the compound represented by formula (I) (abbreviated as E7386 in the figure) and a KRAS inhibitor in the KRAS G12D mutation-positive human endometrial cancer cell line HEC-1-B.
[Figure 3F] Figure 3F is a graph showing cell growth inhibitory activity given by combined treatment with the compound represented by formula (I) (abbreviated as E7386 in the figure) and a KRAS inhibitor in the KRAS G12C mutation-positive human pancreatic cancer cell line MIA PaCa-2.
[Figure 4A] Figure 4A is a graph showing antitumor effects of combined administration of the compound represented by formula (I) (abbreviated as E7386 in the figure) and a KRAS inhibitor in a xenograft model with the KRAS G12C mutation-positive human colorectal cancer cell line SW837.
[Figure 4B] Figure 4B is a graph showing antitumor effects of combined administration of the compound represented by formula (I) (abbreviated as E7386 in the figure) and a KRAS inhibitor in a xenograft model with the KRAS G12C mutation-positive human pancreatic cancer cell line MIA PaCa-2.
[Figure 4C] Figure 4C is a graph showing antitumor effects of combined administration of the compound represented by formula (I) (abbreviated as E7386 in the figure) and a KRAS inhibitor in a xenograft model with the KRAS G12C mutation-positive human bladder cancer cell line UM-UC-3.
[Figure 4D] Figure 4D is a graph showing antitumor effects of combined administration of the compound represented by formula (I) (abbreviated as E7386 in the figure) and a KRAS inhibitor in a xenograft model with the KRAS G12C mutation-positive human non-small cell lung cancer cell line NCI-H358.

### Description of Embodiments

Embodiments of the present disclosure will be described below. Embodiments shown below are examples for describing the present disclosure, and are not intended to limit the present disclosure thereto. The present disclosure may be implemented in various forms unless the forms depart from the gist of the present disclosure.

In the present disclosure, "pharmaceutically acceptable salts" are not particularly limited as long as they form a salt with the compound represented by formula (I), and examples thereof include inorganic salts, organic salts, inorganic basic salts, organic basic salts, and acidic or basic amino acid salts.

Examples of the inorganic salts include hydrochlorides, hydrobromides, sulfates, nitrates, and phosphates, and examples of the organic acid salts include carboxylates such as acetates, succinates, fumarates, maleates, tartrates, citrates, lactates, stearates, benzoates, and mandelates, and sulfonates such as methanesulfonates, ethanesulfonates, p-toluenesulfonates, and benzenesulfonates.

Examples of the inorganic basic salts include alkali metal salts such as sodium salts and potassium salts, alkaline earth metal salts such as calcium salts and magnesium salts, aluminum salts, and ammonium salts, and examples of the organic basic salts include diethylamine salts, diethanolamine salts, meglumine salts, and N,N'-dibenzyl-ethylenediamine salts.

Examples of the acidic amino acid salts include aspartates and glutamates, and examples of the basic amino acid salts include arginine salts, lysine salts, and ornithine salts.

The compound represented by formula (I) is (6 S,9aS)-N-benzyl-8-((6-(3-(4-ethylpiperazin-1-yl)azetidin-1-yl)pyridin-2-yl) methyl)-6-((2-fluoro-4-hydroxyphenyl)methyl)-4,7-dioxo-2-(prop-2-en-1-yl)-octahydro-1H-pyrazino[2,1-c][1,2,4]triazine-1-carboxamide, and the IUPAC name is (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydr o-2H-pyrazino[2,1-c][1,2,4]triazine-1(6H)-carboxamide. Herein, the compound represented by formula (I) is also referred to as "E7386".

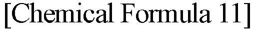

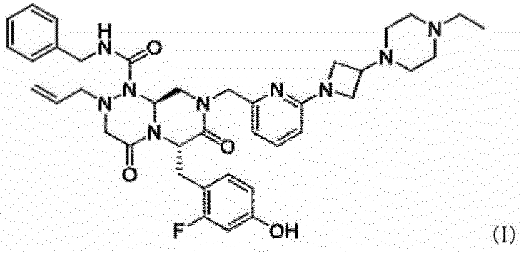

The compound represented by formula (I) can be produced by a method described in U. S. Patent No. 9174998 or U. S. Patent No. 10259817, and exhibits pharmacological activities including antitumor activity.

In the present disclosure, an embodiment of "the compound represented by formula (I) or a pharmaceutically acceptable salt thereof" is, for example, the compound represented by formula (I), that is, (6S,9aS)-N-benzyl-8-({6-[3-(4-ethylpiperazin-1-yl)azetidin-1-yl]pyridin-2-yl} methyl)-6-(2-fhioro-4-hydroxybenzyl)-4,7-dioxo-2-(prop-2-en-1-yl)hexahydr o-2H-pyrazino[2,1-c][1,2,4]triazine-1(6H)-carboxamide.

The term "pharmaceutical composition" or "therapeutic agent" in the present disclosure is understood to mean a substance that causes a desired effect in a tissue, an animal, a mammal, a human, or another subject.

In the present disclosure, the term "treating" and derivatives thereof mean a medical treatment. For specific condition, the term treating means (1) ameliorating the condition or one or more biological signs for the condition, (2) interrupting (a) one or more points in a biological cascade that leads to or causes the condition or (b) one or more biological signs for the condition, (3) mitigating one or more of symptoms, effects, or side effects associated with the condition or one or more of symptoms, effects, or side effects associated with the condition or treatment therefor, or (4) delaying the progression of the condition or one or more biological signs for the condition.

In the present disclosure, the term "therapeutically effective amount" means an amount effective for inducing a desired biological reaction or pharmaceutical reaction in a tissue system, an animal, or a human. In an embodiment, the term therapeutically effective amount means an amount effective for achieving a desired therapeutic outcome (e.g., improvement or prevention of a symptom, or extension of survival) with a necessary dosage within a necessary period. In an embodiment, a therapeutically effective amount is an amount that does not exceed a maximum tolerated dose.

In the present disclosure, the term "patient" refers to a mammal, in particular, a human affected by or suspected to be affected by tumor. The term mammal refers to a mammalian animal such as a guinea pig, a mouse, a rat, a gerbil, a cat, a rabbit, a dog, a bovine, a goat, a sheep, a horse, a monkey, a chimpanzee, and a human. In a certain embodiment, the patient is a human. The pharmaceutical composition, therapeutic agent, or method of the present disclosure is particularly useful for treating a human patient for tumor.

The terms "a", "an", and "the" and similar words used in the context of the present disclosure (especially in the context of claims) are interpreted as encompassing both singular and plural forms, unless otherwise indicated herein or clearly inconsistent with the context.

The "pharmaceutical composition" or "therapeutic agent" of the present disclosure can be administered, for example, through injection (intravenous injection, intraarterial injection, local injection), oral administration, nasal administration, transdermal administration, pulmonary administration, and eye drop administration. Example of the injection include intravenous injection, subcutaneous injection, intradermal injection, intraarterial injection, and local injection to a target cell or organ. Examples of the dosage form of the pharmaceutical composition or therapeutic agent in oral administration include a tablet, a powder, a granule, a syrup, a capsule, and an oral solution. Examples of the dosage form of the pharmaceutical composition in parenteral administration include an injection, a drip infusion, an eye drop, an ointment, a suppository, a suspension, a cataplasm, a lotion, an aerosol, and a plaster, and the dosage form is an injection or a drip infusion in an embodiment. The pharmaceutical composition according to the present disclosure can be formulated, for example, by a method described in the 18th revised Japanese Pharmacopeia (JP), the United States Pharmacopeia (USP), or the European Pharmacopeia (EP).

In an embodiment of the present disclosure, the compound represented by the formula (I) or a pharmaceutically acceptable salt thereof is orally administered to a patient. In another embodiment, the pharmaceutical composition or therapeutic agent comprising the compound represented by formula (I) or a pharmaceutically acceptable salt thereof is for oral administration.

In an embodiment, the pharmaceutical composition or therapeutic agent according to the present disclosure can further contain a pharmaceutically acceptable carrier.

Examples of the pharmaceutically acceptable carrier include a liquid or solid filler, a diluting agent, an excipient, a production aid, and a solvent-encapsulating material.

In the present disclosure, the two objects to be administered in combination can be administered simultaneously or at different times. The phrase "administered simultaneously" in "administered simultaneously or at different times" means that the two objects to be administered in combination are administered at the same time or at substantially the same time through the same route of administration (simultaneous administration), or administered at the same time or at substantially the same time through different routes of administration (separate administration). Here, the meaning of "administered at the same time" also includes the case that the two objects are administered as one formulation. In the present disclosure, the phrase "administered at different times" in "administered simultaneous or at different times" means that the two objects to be administered in combination are administered at different times through the same route of administration or different routes of administration (sequential administration). More specifically, the phrase means that the completion of administration of one of the two objects to be administered in combination is followed by the initiation of administration of the other. The meaning of "administered at different times" also includes the case that different frequencies of administration or different periods of administration are employed for a dosing regimen for the two objects.

The dosage of the compound represented by formula (I) or a pharmaceutically acceptable salt thereof in monotherapy significantly depends on the type of target disease, the age, sex, and body weight of a patient, degree of symptoms, and so on. Although the dosage of the compound represented by formula (I) or a pharmaceutically acceptable salt thereof is not limited, in oral administration to an adult (body weight: 60 kg) or a child, the dose in terms of the compound represented by formula (I) is typically 0.1 to 5000 mg, 0.5 to 3000 mg, or 1.0 to 1000 mg per day, and 100 mg to 300 mg per day in an embodiment. This can be administered once in one or more days, or in two to six portions in one day. The dosage and dosing regimen can be modified if one or more additional chemotherapeutics are used. The dosing regimen can be determined by a physician treating a specific patient. In an embodiment, for example, a dosage of 80 mg to 120 mg in terms of the compound represented by formula (I) per administration is orally administered to a human subject twice per day. A specific dose, route of administration, frequency of administration, administration cycle, and so on for the compound represented by formula (I) or a pharmaceutically acceptable salt thereof are appropriately determined in view of the type of target disease, the age, sex, and body weight of a patient, degree of symptoms, other drugs to be administered in combination, and so on.

The RAS inhibitor in the present disclosure may contain any compound or biomolecule that (a) binds to a biomolecule (e.g., protein, nucleic acid) having RAS to inhibit the functions, (b) binds to a biomolecule (e.g., protein, nucleic acid) having RAS to decompose the biomolecule, (c) suppresses expression of a biomolecule (e.g., protein, nucleic acid) having RAS, (d) inhibits the protein-protein interaction between RAS and a guanine nucleotide exchange factor (e.g., SOS1, SOS2) to inhibit the GDP-GTP exchange reaction of RAS, or (e) functions as a vaccine to stimulate immunity to mutant RAS.

Examples of the RAS inhibitor in an embodiment in the present disclosure include sotorasib (AMG 510), adagrasib (MRTX849), MRTX1133, BI-3406, BI 1701963, RG6330, LY3537982, JDQ443, RMC-6291, RMC-6236, LUNA18, ARS-1620, JNJ-74699157, GDC-6036, iExosomes, D-1553, mRNA5671, BI 2852, SCH-54292, TLN-4601, salirasib, deltarasin, JAB-21822, BI 1823911, MK-1084, ELI-002, SDGR5, JAB-22000, and ASP3082.

Sotorasib (also referred to as AMG 510) is a compound having the following structure: 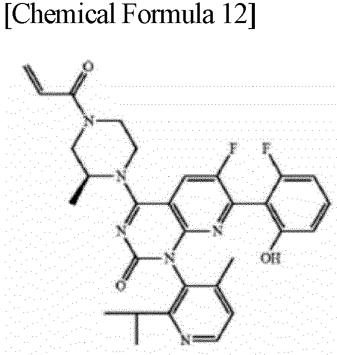 with the chemical name of 6-fluoro-7-(2-fhioro-6-hydroxyphenyl)-1-(4-methyl-2-(2-propanyl)-3-pyridin yl)-4-((2S)-2-methyl-4-(2-propenoyl)-1-piperazinyl)pyrido[2,3-d]pyrimidin-2( 1H)-one. The structural formula of sotorasib (AMG 510) and a production method therefor are described from line 10 of Column 411 to line 19 of Column 415 in U. S. Patent No. 10519146, the contents of which are incorporated herein by reference.

Adagrasib (also referred to as MRTX849) is a compound having the following structure: 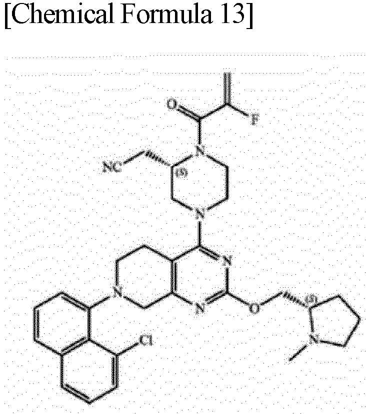 with the chemical name of 2-[(2S)-4-[7-(8-chloro-1-naphthyl)-2-[[(2S)-1-methylpyrrolidin-2-yl]methoxy ]-6,8-dihydro-SH-pyrido [3,4-d]pyrimidin-4-yl]-1-(2-fluoroprop-2-enoyl)piper azin-2-yl]acetonitrile. The structural formula of adagrasib (MRTX849) and a production method therefor are described from line 32 of Column 1286 to line 17 of Column 1288 in U. S. Patent No. 10689377, the contents of which are incorporated herein by reference.

MRTX1133 is a compound having the following structure: 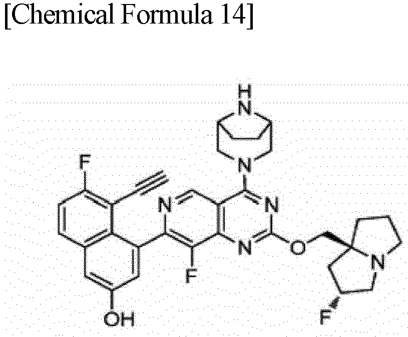 with the chemical name of 4-(4-((1R,5S)-3,8-diazabicyclo[3.2.1]octan-3-yl)-8-fluoro-2-(((2R,7aS)-2-fluo rohexahydro-1H-pyrrolidin-7a-yl)methoxy)pyrodo[4,3-d]pyrimidin-7-yl)-5-et hynyl-6-fluoronaphthalen-2-ol. The structural formula of MRTX1133 and a production method therefor are described in WO 2021/041671.

The pharmacological activity of BI 1701963 has been reported in AACR Annual Meeting 2021#CT210; 2021 Apr 10-15 and May 17-21 (Abstract https://doi.org/10.1158/1538-7445.AM2021-CT210).

The structural formula of BI-3406 and a production method therefor are described in U. S. Patent No. 10898487, the contents of which are incorporated herein by reference.

RG6330 is a compound identical to GDC-6036. The structural formula of RG6330 and a production method therefor are described in U. S. Patent No. 11236068, the contents of which are incorporated herein by reference.

The structural formula of JDQ443 and a production method therefor are described in WO 2021/124222.

The structural formula of ARS-1620 and a production method therefor are described in U. S. Patent No. 10370386, the contents of which are incorporated herein by reference.

The pharmacological activity of iExosomes has been reported in Nature volume 546, pages 498-503 (2017).

The structural formula of D-1553 and a production method therefor are described in U. S. Patent No. 11091481, the contents of which are incorporated herein by reference.

The structural formula of BI 2852 and a production method therefor are described in WO 2020/173935.

The structural formula of SCH-54292 and a production method therefor have been reported in Bioorganic & Medicinal Chemistry, Volume 5, Issue 1, January 1997, Pages 125-133.

The structural formula of TLN-4601 and a production method therefor are described in U. S. Patent No. 7101872, the contents of which are incorporated herein by reference.

The structural formula of salirasib and a production method therefor are described in U. S. Reissue Patent No. RE39,682, the contents of which are incorporated herein by reference.

The structural formula of deltarasin and a production method therefor are described in U. S. Patent No. 9861623, the contents of which are incorporated herein by reference.

In the present disclosure, examples of the RAS inhibitor include sotorasib (AMG 510), adagrasib (MRTX849), MRTX1133), BI-3406, BI 1701963, RG6330, LY3537982, JDQ443, RMC-6291, RMC-6236, LUNA18, ARS-1620, JNJ-74699157, GDC-6036, iExosomes, D-1553, mRNA5671, BI 2852, SCH-54292, TLN4601, Salirasib, Deltarasin, JAB-21822, BI 1823911, MK-1084, ELI-002, SDGR5, JAB-22000, and ASP3082; the RAS inhibitor in an embodiment is any one of sotorasib (AMG 510), adagrasib (MRTX849), MRTX1133, BI-3406, BI 1701963, RG6330, LY3537982, JDQ443, RMC-6291, RMC-6236, LUNA18, ARS-1620, JNJ-74699157, GDC-6036, iExosomes, mRNA-5671, JAB-21822, BI 1823911, MK-1084, ELI-002, SDGR5, JAB-22000, and ASP3082; and the RAS inhibitor in a particular embodiment is any one of sotorasib (AMG 510), adagrasib (MRTX849), and BI 1701963.

In an embodiment of the present disclosure, the RAS inhibitor is a KRAS inhibitor, an NRAS inhibitor, or an BRAS inhibitor. In another embodiment of the present disclosure, the RAS inhibitor is a KRAS inhibitor.

In the present disclosure, the KRAS inhibitor is an inhibitor that (i) binds to KRAS to block downstream signals due to RAS, or (ii) inhibits the interprotein interaction between KRAS and a guanine nucleotide exchange factor (e.g., SOS1, SOS2). In the case of (i), the inhibitor is an RAS-on inhibitor if the inhibitor binds to a GTP-binding mutant KRAS protein, and the inhibitor is an RAS-off inhibitor if the inhibitor binds to GDP-binding mutant KRAS protein.

In an embodiment of the present disclosure, the KRAS inhibitor is any one of sotorasib, adagrasib, MRTX1133, BI-3406, BI 1701963, RG6330, LY3537982, JDQ443, RMC-6291, RMC-6236, LUNA18, ARS-1620, JNJ-74699157, GDC-6036, iExosomes, mRNA-5671, JAB-21822, BI 1823911, MK-1084, ELI-002, SDGR5, JAB-22000, andASP3082.

The RAS-on inhibitor in an embodiment of the present disclosure is any one of MRTX1133, RMC-6291, and RMC-6236. The RAS-off inhibitor in an embodiment of the present disclosure is any one of sotorasib, adagrasib, MRTX1133, RG6330, LY3537982, JDQ443, ARS-1620, JNJ-74699157, JAB-21822, BI 1823911, MK-1084, and JAB-22000.

An example of the KRAS inhibitor includes a KRAS G12C inhibitor, a KRAS G12D inhibitor, and a pan-KRAS inhibitor, and, in an embodiment, for example, a KRAS G12C inhibitor. Here, the KRAS G12C inhibitor is an inhibitor that binds to a KRAS mutant (e.g., protein, nucleic acid) having a mutation substituting Gly at position 12 of KRAS with Cys to block downstream signals due to the mutant KRAS, and the KRAS G12D inhibitor is an inhibitor that binds to a KRAS mutant (e.g., protein, nucleic acid) having a mutation substituting Gly at position 12 of KRAS with Asp to block downstream signals due to the mutant KRAS. The pan-KRAS inhibitor is (a) an inhibitor that binds to KRAS (e.g., protein, nucleic acid), irrespective of the presence or absence of a mutation, the position of a mutation, and an amino acid after mutation, to block downstream signals due to RAS, or (b) an inhibitor that binds to a guanine nucleotide exchange factor (e.g., SOS1, SOS2) for KRAS to inhibit the interprotein interaction between KRAS and the guanine nucleotide exchange factor, thereby blocking downstream signals due to RAS.

In an embodiment, the KRAS G12C inhibitor is any one of sotorasib (AMG 510), adagrasib (MRTX849), RG6330, LY3537982, JDQ443, RMC-6291, JAB-21822, BI 1823911, and MK-1084, and, in another embodiment, the KRAS G12C inhibitor is any one of sotorasib (AMG 510) and adagrasib (MRTX849). In an embodiment, the KRAS G12D inhibitor is any one of MRTX1133 and JAB-22000. In an embodiment, the pan-KRAS inhibitor is any one of BI-3406, BI 1701963, LUNA18, SDGR5, and RMC-6236.

The RAS inhibitor according to the present disclosure can be administered, for example, through injection (intravenous injection, intraarterial injection, local injection), oral administration, nasal administration, transdermal administration, pulmonary administration, and ophthalmic administration. Example of the injection include intravenous injection, subcutaneous injection, intradermal injection, intraarterial injection, and local injection to a target cell or organ. Examples of the dosage form of the RAS inhibitor in oral administration include a tablet, a powder, a granule, a syrup, a capsule, and an oral solution. Examples of the dosage form of the RAS inhibitor in parenteral administration include an injection, a drip infusion, an eye drop, an ointment, a suppository, a suspension, a cataplasm, a lotion, an aerosol, and a plaster, and the dosage form is an injection or a drip infusion in an embodiment. The RAS inhibitor according to the present disclosure can be formulated, for example, by a method described in the Japanese Pharmacopeia (JP), Eighteenth Edition, the United States Pharmacopeia (USP), or the European Pharmacopeia (EP).

Although the dosage of the RAS inhibitor is not limited, in oral administration to an adult (body weight: 60 kg) or a child, the dose is 0.1 to 5000 mg, 0.5 to 3000 mg, or 1.0 to 1000 mg per day. This can be administered once in one or more days, or in two to six portions in one day. The dosage and dosing regimen can be modified if one or more additional chemotherapeutics are used. The dosing regimen can be determined by a physician treating a specific patient. In the case that sotorasib alone is administered as the RAS inhibitor, for example, a dose selected from the group consisting of 960 mg, 480 mg, 240 mg, and 120 mg per day is orally administered. A specific dosage, route of administration, frequency of administration, administration cycle, and so on for the RAS inhibitor are appropriately determined in view of the type of target disease, the age, sex, and body weight of a patient, degree of symptoms, other drugs to be administered in combination, and so on.

The tumor to be treated in the present disclosure is, for example, a solid tumor. In an example, the solid tumor as the tumor to be treated is non-small cell lung cancer, colorectal cancer, pancreatic cancer, mesothelioma, endometrial cancer (e.g., cervical cancer, corpus uteri cancer), ovarian cancer, bladder cancer, bile duct cancer, gastric cancer, breast cancer, small cell lung cancer, testis cancer, small intestine cancer, appendix cancer, or neuroendocrine tumor; in an embodiment, the solid tumor is at least one selected from non-small cell lung cancer, colorectal cancer, pancreatic cancer, ovarian cancer, and endometrial cancer; in another embodiment, the solid tumor is at least one selected from non-small cell lung cancer, colorectal cancer, pancreatic cancer, endometrial cancer, bile duct cancer, and bladder cancer; and, in a particular embodiment, the solid tumor is at least one selected from non-small cell lung cancer and colorectal cancer. In an embodiment, the tumor to be treated is at least one selected from non-small cell lung cancer, colorectal cancer, pancreatic cancer, ovarian cancer, bladder cancer, bile duct cancer, gastric cancer, breast cancer, small cell lung cancer, and endometrial cancer (e.g., cervical cancer, corpus uteri cancer). In another embodiment, the tumor to be treated is at least one selected from non-small cell lung cancer, colorectal cancer, pancreatic cancer, ovarian cancer, and bladder cancer; and, in still another embodiment, the tumor to be treated is at least one selected from colorectal cancer, pancreatic cancer, ovarian cancer, bladder cancer, bile duct cancer, gastric cancer, breast cancer, small cell lung cancer, and endometrial cancer (e.g., cervical cancer, corpus uteri cancer).

An embodiment of the present disclosure is, for example, a pharmaceutical composition for treating a tumor (or a therapeutic agent for a tumor), the pharmaceutical composition comprising the compound represented by formula (I), wherein the pharmaceutical composition is administered in combination with sotorasib, and the tumor to be treated is non-small cell lung cancer.

Another embodiment of the present disclosure is, for example, a pharmaceutical composition for treating a tumor (or a therapeutic agent for a tumor), the pharmaceutical composition comprising the compound represented by formula (I), wherein the pharmaceutical composition is administered in combination with sotorasib, and the tumor to be treated is at least one selected from non-small cell lung cancer, colorectal cancer, pancreatic cancer, and bladder cancer, and, in a still particular embodiment, the tumor to be treated is at least one selected from non-small cell lung cancer and colorectal cancer.

An embodiment of the present disclosure is, for example, a pharmaceutical composition for treating a tumor (or a therapeutic agent for a tumor), the pharmaceutical composition comprising the compound represented by formula (I), wherein the pharmaceutical composition is administered in combination with adagrasib, and the tumor to be treated is non-small cell lung cancer.

Another embodiment of the present disclosure is, for example, a pharmaceutical composition for treating a tumor (or a therapeutic agent for a tumor), the pharmaceutical composition comprising the compound represented by formula (I), wherein the pharmaceutical composition is administered in combination with adagrasib, and the tumor to be treated is at least one selected from non-small cell lung cancer and colorectal cancer.

An embodiment of the present disclosure is, for example, a pharmaceutical composition for treating a tumor (or a therapeutic agent for a tumor), the pharmaceutical composition comprising the compound represented by formula (I), wherein the pharmaceutical composition is administered in combination with MRTX1133, and the tumor to be treated is, in an embodiment, at least one selected from non-small cell lung cancer, colorectal cancer, and pancreatic cancer. In another embodiment, the tumor to be treated with the pharmaceutical composition for treating a tumor (or a therapeutic agent for a tumor), the pharmaceutical composition comprising the compound represented by formula (I), wherein the pharmaceutical composition is administered in combination with MRTX1133, is at least one selected from endometrial cancer and bile duct cancer.

A method of treating a tumor, wherein the compound represented by formula (I) or a pharmaceutically acceptable salt thereof and the RAS inhibitor are administered in combination, is provided as an embodiment of the present disclosure. In a certain embodiment, the compound represented by formula (I) or a pharmaceutically acceptable salt thereof is orally administered.

An embodiment of the present disclosure is, for example, a method of treating a tumor, wherein a therapeutically effective amount of the compound represented by formula (I) and sotorasib are orally administered to a patient in combination, and the tumor to be treated is non-small cell lung cancer.

An embodiment of the present disclosure is, for example, a method of treating a tumor, wherein a therapeutically effective amount of the compound represented by formula (I) and sotorasib are orally administered to a patient in combination, and the tumor to be treated is at least one selected from non-small cell lung cancer, colorectal cancer, pancreatic cancer, and bladder cancer, and, in a still particular embodiment, the tumor to be treated is at least one selected from non-small cell lung cancer and colorectal cancer.

An embodiment of the present disclosure is, for example, a method of treating a tumor, wherein a therapeutically effective amount of the compound represented by formula (I) and adagrasib are orally administered to a patient in combination, and the tumor to be treated is non-small cell lung cancer.

Another embodiment of the present disclosure is, for example, a method of treating a tumor, wherein a therapeutically effective amount of the compound represented by formula (I) and adagrasib are orally administered to a patient in combination, and the tumor to be treated is at least one selected from non-small cell lung cancer and colorectal cancer.

An embodiment of the present disclosure is, for example, a method of treating a tumor, wherein a therapeutically effective amount of the compound represented by formula (I) and MRTX1133 are orally administered to a patient in combination, and the tumor to be treated is at least one selected from non-small cell lung cancer, colorectal cancer, and pancreatic cancer. Another embodiment is, for example, a method of treating a tumor, wherein a therapeutically effective amount of the compound represented by formula (I) and MRTX1133 are orally administered to a patient in combination, and the tumor to be treated is at least one selected from endometrial cancer and bile duct cancer.

The terms "comprise" and "include/contain" are intended to be open-ended and comprehensive except for the case that it is obvious from the context that the terms should be otherwise understood, and the terms do not exclude additional properties that are not shown and encompass the closed terms "consist of' or "essentially consist of".

All the terms used herein (including technical terms and scientific terms) have the same meanings as widely understood by those skilled in the art to which the present invention belongs, unless otherwise defined. Unless other definitions are explicitly stated, the terms used herein should be understood to have meanings consistent with those in the present specification and the related technical field, and should not be interpreted to have idealized or excessively formal meanings.

### Examples

### [Example 1]

### Antitumor Effect of Combined Treatment of Compound Represented by Formula (I) and KRAS Inhibitor in KRAS G12C Mutation-Positive Human Non-Small Cell Lung Cancer Cell Line NCI-H358

With the KRAS G12C mutation-positive human non-small cell lung cancer cell line NCI-H358 (ATCC Cat. No. CRL-5807), combined treatment of the compound represented by formula (I) and sotorasib (manufactured by Angene International Limited) was evaluated on growth inhibitory activity. Cells were cultured in RPMI1640 medium (manufactured by FUJIFILM Wako Pure Chemical Corporation) containing 10% v/v fetal bovine serum (FBS). Cells were seeded in 96-well plates at 2 × 10³ cells/well, and cultured at 5% CO₂ and 37°C for 24 hours. Thereafter, the compound represented by formula (I) (TOP: 370 nM, common ratio: 3) and sotorasib (TOP: 1 µM, common ratio: 3) were prepared, and culture was performed with treatment of sotorasib alone or with combined treatment of sotorasib and the compound represented by formula (I) for 72 hours. At that time, DMSO stocks of the compound represented by formula (I) and sotorasib was diluted with medium to prepare the solution at specific concentrations. For the treatment of sotorasib alone, medium was added instead of drug solution of the compound represented by formula (I). After the culture for 72 hours, the cells were fixed with 10% trichloroacetic acid. Subsequently, the fixed cells were stained with 0.4% (wt/vol) sulforhodamine B (SRB) dissolved in 1% acetic acid, and the absorbance at 515 nm was measured with a plate reader (manufactured by PerkinElmer, Inc.). The measurement values were defined as the relative cell counts. The cell counts of monotreatment with the compound represented by formula (I) at each concentration were defined as 100%, and GI₅₀ was calculated by using the cell count before and after the treatment for 72 hours.

In the experiment, the compound represented by formula (I) was dissolved in 0.1 M hydrochloric acid, and sotorasib wasdissolved in 1% Tween80 (manufactured by Sigma-Aldrich Co. LLC)/2% HPMC (manufactured by FUJIFILM Wako Pure Chemical Corporation)/97% distilled water (manufactured by Otsuka Pharmaceutical Factory, Inc.) before use.

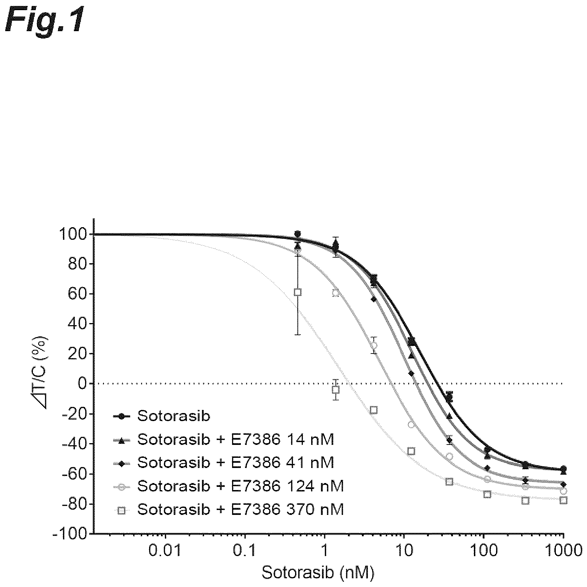

The results of growth inhibition curves and GI₅₀ were shown in Table 1 and Figure 1. In Table 1 and Figure 1, E7386 indicates the compound represented by formula (I). In Figure 1, the abscissa shows concentrations of sotorasib, and the ordinate shows ΔT/C. Measurement results were expressed as mean ± standard error (SEM) of ΔT/C in Figure 1. ΔT/C indicates the cell growth rate in the condition where sotorasib alone is treated (sotorasib group) to the cell growth rate in the condition where sotorasib and the compound represented by formula (I) are treated in combination. The results showed that combined treatment of the compound represented by formula (I) and sotorasib exhibited significant growth inhibitory action as compared with sotorasib alone (sotorasib group) in KRAS G12C mutation-positive human non-small cell lung cancer cell line NCI-H358.

**[Table 1]**

| Group | GI₅₀ (nM) |
|---|---|
| Sotorasib group | 7.79 |
| Sotorasib + E7386 14 nM group | 6.63 |
| Sotorasib + E7386 41 nM group | 5.07 |
| Sotorasib + E7386 123 nM group | 2.10 |
| Sotorasib + E7386 370 nM group | 0.45 |

### [Example 2]

### Antitumor Effect of Combined Administration of Compound Represented by Formula (I) and KRAS Inhibitor in Xenograft Model with KRAS G12C Mutation-Positive Human Non-Small Cell Lung Cancer Cell Line NCI-H358

Matrigel/HBSS (50:50) was added to a KRAS G12C mutation-positive human non-small cell lung cancer cell line, NCI-358 cells, to prepare a cell suspension of 8 × 10⁶ cells. The cell suspension was subcutaneously transplanted into nude mice (CAnN.Cg-Foxn1 nu/CrlCrlj, from Charles River Laboratories Japan, Inc.) at the body flank. Six mice were used per group. The date of transplantation was defined as day 1. From day 7 after the transplantation, the compound represented by formula (I) (25 mg/kg, once per day, for 14 days, oral administration) and sotorasib (100 mg/kg, once per day, for 14 days, oral administration) were administered alone or in combination to a monotherapy group or a combined administration group. No drug was administered to the control group.

Before the administration, the compound represented by formula (I) was dissolved in 0.1 M hydrochloric acid, and sotorasib was dissolved in 1% Tween80 (manufactured by Sigma-Aldrich Co. LLC)/2% HPMC (manufactured by FUJIFILM Wako Pure Chemical Corporation)/97% distilled water (manufactured by Otsuka Pharmaceutical Factory, Inc.). The date of the initiation of administration was defined as day 1. On days 4, 8, 11, and 15, the long diameter and short diameter of tumors generated in each mouse were measured with a Digimatic Caliper (manufactured by Mitutoyo Corp.).

The tumor volume was calculated by the following formula. Tumor volume (mm3) = Tumor long diameter (mm) × Tumor short diameter2 (mm2) / 2

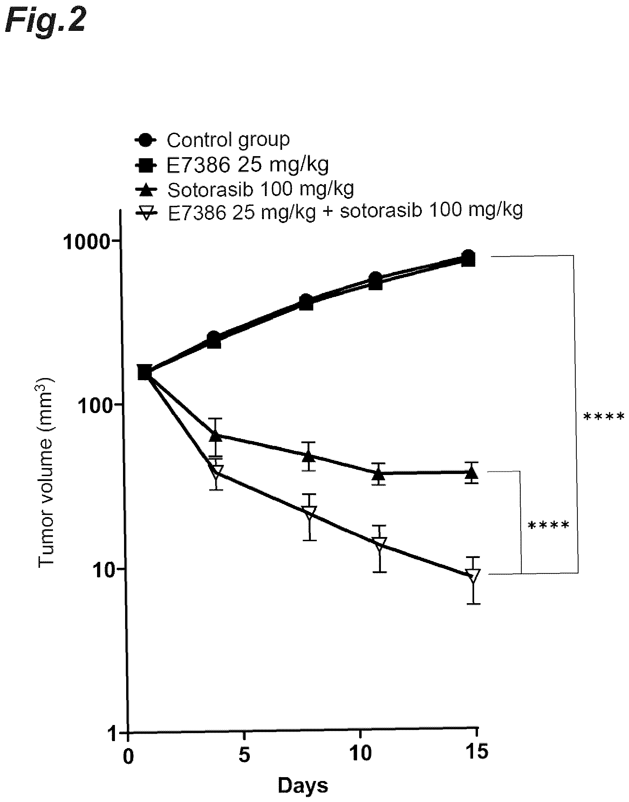

The results of measurement of tumor volume for the groups were shown as means and standard errors (SEM) in Table 2 and Figure 2. Numbers in Table 2 each indicate mean ± standard error (SEM) of tumor volume.

The results showed that the combined administration of the compound represented by formula (I) and sotorasib exhibited significant antitumor effect in the xenograft model with the KRAS G12C mutation-positive human non-small cell lung cancer cell line NCI-H358. In Table 2 and Figure 2, E7386 indicates the compound represented by formula (I). **** in Figure 2 indicates that combined administration of the compound represented by formula (I) and sotorasib statistically significantly inhibited tumor growth compared with administration of each drug alone (****: p < 0.0001; Repeated measures ANOVA followed by Dunnett's type multiple comparison after logarithmic transformation).

**[Table 2]**

| Group | Day 4 | Day 8 | Day 11 | Day 15 |
|---|---|---|---|---|
| Control group | 250.3±8.1 | 413.4±31.1 | 555.9±30.2 | 746+52.5 |
| E7386 25 mg/kg group | 236.7±13.2 | 396.8±27.6 | 519.9±34.1 | 714.4+55.5 |
| Sotorasib 100 mg/kg group | 64.3±15.2 | 47.4±8.6 | 36.2±4.9 | 36.2±4.8 |
| E7386 25 mg/kg + Sotorasib 100 mg/kg group | 37.8±7.4 | 20.8±6.0 | 13.2±3.8 | 8.3±2.4 |

### [Example 3]

### Cell Growth Inhibitory Activity Given by Combined Treatment with Compound Represented by Formula (I) and KRAS Inhibitor (Sotorasib, Adagrasib, MRTX1133, or BI3406) in KRAS G12C Mutation-Positive Human Pancreatic Cancer Cell Line MIA PaCa-2, KRAS G12C Mutation-Positive Human Bladder Cancer Cell Line UM-UC-3, KRAS G12C Mutation-Positive Human Non-Small Cell Lung Cancer Cell Line NCI-H358, KRAS G12D Mutation-Positive Human Bile Duct Cancer Cell Line SNU-869, and KRAS G12D Mutation-Positive Human Endometrial Cancer Cell Line HEC-1-B

With different KRAS mutation-positive cell lines, combined treatment of the compound represented by formula (I) and a KRAS inhibitor was evaluated on growth inhibitory activity. As the KRAS inhibitor, sotorasib (manufactured by Angene International Limited), adagrasib (manufactured by Amadis Chemical Company Limited), or MRTX1133 (manufactured by WuXi AppTec) was used. The providers of culture media for the cell lines were shown in Table 3. Cells were seeded in 96-well flat-bottomed ultra-low adsorption plate (manufactured by Corning Incorporated) at 5000 cells/well, and cultured at 5% CO2 and 37°C for 1 day. Thereafter, the compound represented by formula (I) (TOP: 333 nM, common ratio: 3) and any one of the three KRAS inhibitors: sotorasib (TOP: 0.2-1 µM, common ratio: 5), adagrasib (TOP: 1 µM, common ratio: 5), MRTX1133 (TOP: 1 µM, common ratio: 5), or BI3406 (TOP: 20 µM, common ratio: 5) were prepared, and culture was performed with the treatment of the KRAS inhibitor alone or with combined treatment of the KRAS inhibitor and the compound represented by formula (I) for 5 days. The drug solutions were prepared by diluting their DMSO stocks with medium to specific concentrations, and added in such a manner that the volume of the culture medium reached 100 µL/well after addition of the drugs. After the culture for 5 days, 30 µL of CellTiter-Glo 3D Reagent (manufactured by Promega Corporation) was added to each well and reacted under shading for 30 minutes, and 50-µL portions were then transferred into a separate white 96-well plate (manufactured by Greiner Bio-One International GmbH), and the emission intensity of luciferase was measured with a plate reader (manufactured by PerkinElmer, Inc.). The measurement values were defined as the relative cell counts. The cell counts of monotreatment with the compound represented by formula (I) at each concentration were defined as 100%, and GI₅₀ was calculated by using the cell counts before and after the treatment for 5 days.

**[Table 3]**

| Cell line | Supplier | Catalog No. | Culture medium (manufacturer) |
|---|---|---|---|
| MIA PaCa-2 | ATCC | CRL-1420 | DMEM (high-glucose) medium (Fujifilm Wako) containing 10% FBS (Sigma) |
| UM-UC-3 | DS Pharma Biomedical | EC96020936-FC | EMEM medium (Fujifilm Wako) containing 10% FBS (Sigma) |
| NCI-H358 | ATCC | CRL-5807 | RPMI1640 medium (Fujifilm Wako) containing 10% FBS (Sigma) |
| SNU-869 | Creative Bioarray | CSC-C9711L | RPMI1640 medium (Fujifilm Wako) containing 10% FBS (Sigma) |
| HEC-1-B | HSRRB (JCRB) | JCRB1193 | EMEM medium (Fujifilm Wako) containing 10% FBS (Sigma) |
| SW837 | ATCC | CCL-235 | Leibovitz's L-15 medium (Fujifilm Wako) containing 10% FBS (Sigma) |

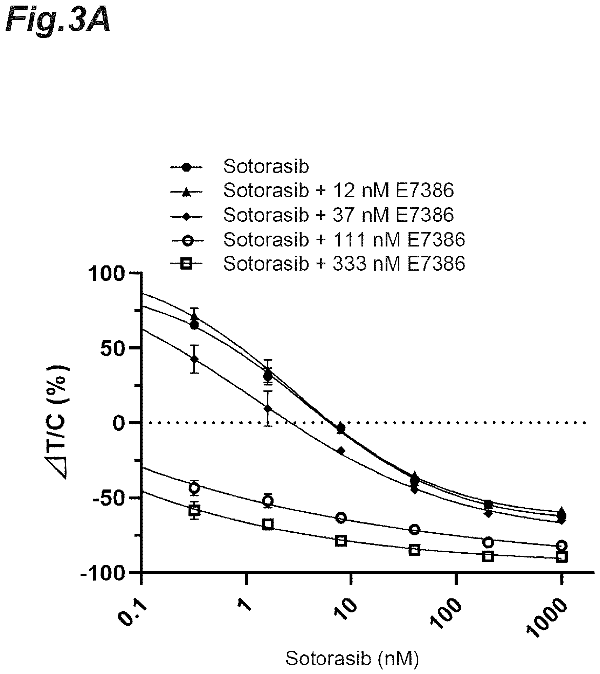

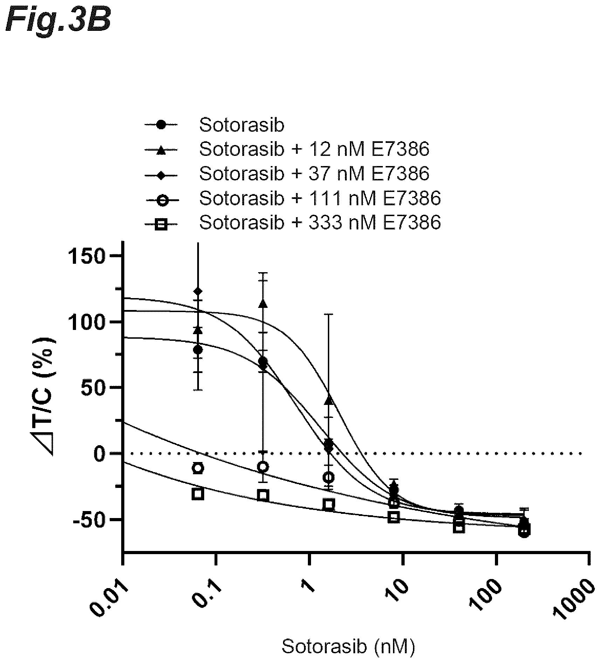

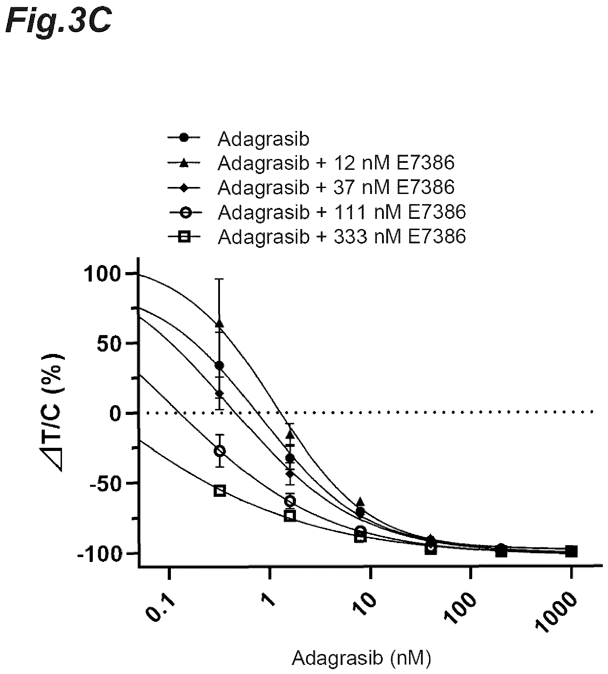

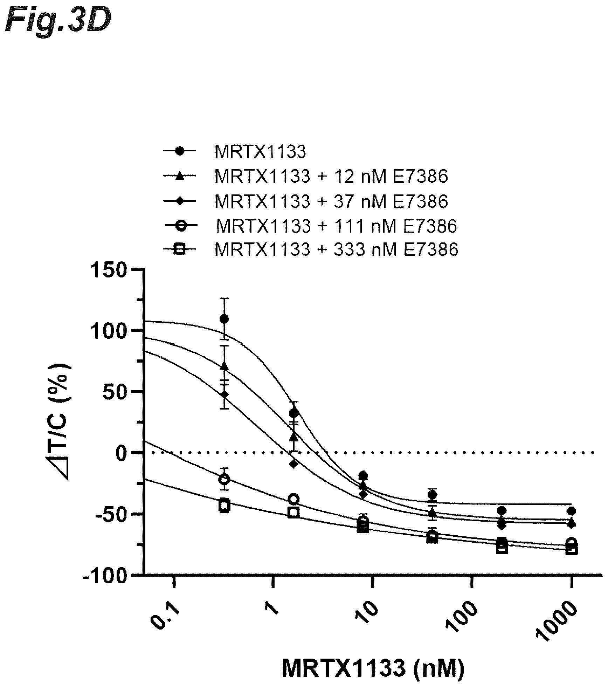

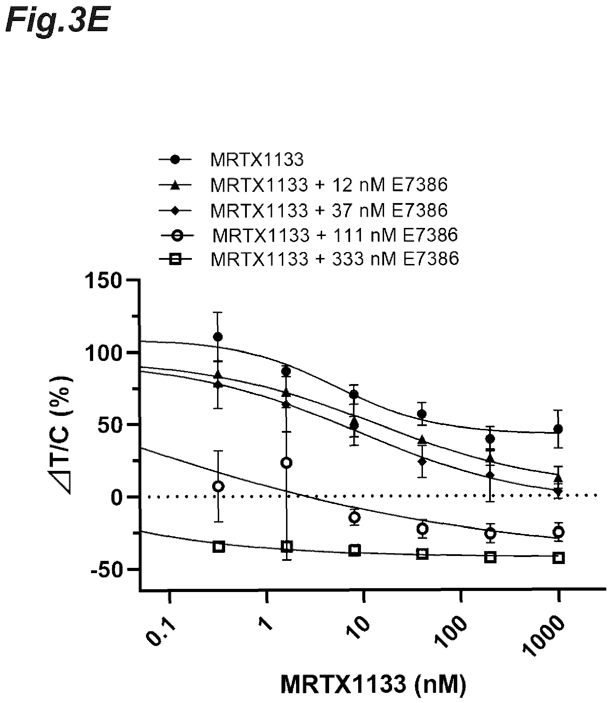

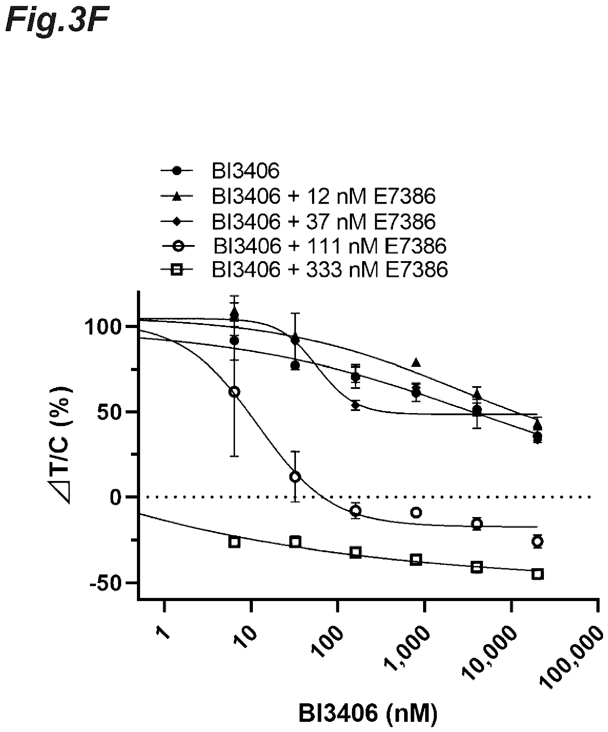

The results as GI₅₀ and growth inhibition curves were shown in Table 4 and Figures 3A to 3F. In Table 4 and Figures 3A to 3F, E7386 indicates the compound represented by formula (I). In each of Figures 3A to 3F, the abscissa shows concentrations of the corresponding KRAS inhibitor, and the ordinate shows ΔT/C. For each condition for drug addition, measurement results of N = 3 (for three wells) were expressed as mean ± standard deviation (SD) of ΔT/C in Figures 3A to 3F. ΔT/C indicates the cell growth rate in the condition where a KRAS inhibitor alone is treated to the cell growth rate in the condition where the KRAS inhibitor and E7386 are treated in combination. The results showed that combined treatment of E7386 and sotorasib exhibited significant growth inhibitory activity compared with sotorasib alone in KRAS G12C mutation-positive human pancreatic cancer cell line MIA PaCa-2 and the KRAS G12C mutation-positive human bladder cancer cell line UM-UC-3 (Figure 3A, Figure 3B). Combined treatment of E7386 and adagrasib exhibited significant growth inhibitory activity compared with adagrasib alone in KRAS G12C mutation-positive human non-small cell lung cancer cell line NCI-H358 (Figure 3C). Combined treatment of E7386 and MRTX1133 exhibited significant growth inhibitory activity compared with MRTX1133 alone in KRAS G12D mutation-positive human bile duct cancer cell line SNU-869 and the KRAS G12D mutation-positive human endometrial cancer cell line HEC-1-B (Figure 3D, Figure 3E). Furthermore, combined treatment of E7386 and BI3406 exhibited significant growth inhibitory activity compared with BI3406 alone in KRAS G12C mutation-positive human pancreatic cancer cell line MIA PaCa-2 (Figure 3F).

**[Table 4]**

| Cell line | Group | GI₅₀ (nM) | Cell line | Group | GI₅₀ (nM) |
|---|---|---|---|---|---|
| MIAPaCa-2 | Sotorasib group | 3.59 | UM-UC-3 | Sotorasib group | 1.30 |
| | Sotorasib + E7386 12 nM group | 2.85 | | Sotorasib + E7386 12 nM group | 2.07 |
| | Sotorasib + E7386 37 nM group | 1.16 | | Sotorasib + E7386 37 nM group | 0.72 |
| | Sotorasib + E7386 111 nM group | 2.48 × 10⁻¹⁴ | | Sotorasib + E7386 111 nM group | 5.21 × 10⁻²⁰ |
| | Sotorasib + E7386 333 nM group | 3.77 × 10⁻⁵ | | Sotorasib + E7386 333 nM group | 4.29 × 10⁻¹⁵ |

| Cell line | Group | GI₅₀ (nM) | Cell line | Group | GI₅₀ (nM) |
|---|---|---|---|---|---|
| NCI-H358 | Adagrasib group | 0.82 | SNU-869 | MRTX1133 group | 1.79 |
| | Adagrasib +E7386 12 nM group | 1.14 | | MRTX1133 + E7386 12 nM group | 1.36 |
| | Adagrasib + E73 86 37 nM group | 0.41 | | MRTX1133 + E7386 37 nM group | 0.69 |
| | Adagrasib + E7386 111 nM group | 0.11 | | MRTX1133 + E7386 111 nM group | 0.022 |
| | Adagrasib + E7386 333 nM group | 0.017 | | MRTX1133 + E7386 333 nM group | 5.69 × 10⁻¹⁵ |

| Cell line | Group | GI₅₀ (nM) | Cell line | Group | GI₅₀ (µM) |
|---|---|---|---|---|---|
| HEC-1B | MRTX1133 group | 5.37 | MIAPaCa-2 | BI3406 group | 3.90 |
| | MRTX1133 + E7386 12 nM group | 13.51 | | BI3406 + E7386 12 nM group | 2.60 |
| | MRTX1133 + E7386 37 nM group | 8.89 | | BI3406 + E7386 37 nM group | 0.058 |
| | MRTX1133 + E7386 111 nM group | 9.09 × 10⁻³ | | BI3406 + E7386 111 nM group | 0.012 |
| | MRTX1133 + E7386 333 nM group | 6.68 × 10⁻¹⁰ | | BI3406 + E7386 333 nM group | 4.35 × 10⁻¹⁹ |

### [Example 4]

### Antitumor effect of Combined Administration of E7386 and KRAS Inhibitor (Sotorasib or Adagrasib) in Xenograft Models with KRAS G12C Mutation-Positive Human Colorectal Cancer Cell Line SW837, KRAS G12C Mutation-Positive Human Pancreatic Cancer Cell Line MIA PaCa-2, KRAS G12C Mutation-Positive Human Bladder Cancer Cell Line UM-UC-3, and KRAS G12C Mutation-Positive Human Non-Small Cell Lung Cancer Cell Line NCI-H358

Experimental conditions for drug efficacy test using mice with different cell lines (suspension media, cell counts for transplantation, lineages of mice, administered drugs, days of initiation of administration, test periods) were shown in Table 5. Each cell line was prepared with suspension medium to reach an intended cell count for transplantation, and the resulting suspension was subcutaneously transplanted into nude mice (6-week-old, female) at the body flank. From the day of initiation of administration shown in Table 5, the compound represented by formula (I) (25 mg/kg, once per day, oral administration) and sotorasib (100 mg/kg, once per day, oral administration) or adagrasib (10 mg/kg, once per day, oral administration) were administered as monotherapy or in combination to a monotherapy group or a combined administration group until the day before the final day of the test period.

Before the administration, E7386 was prepared with 0.1 M hydrochloric acid (manufactured by FUJIFILM Wako Pure Chemical Corporation), sotorasib was prepared with 1% Tween80 (Tokyo Chemical Industry Co., Ltd.)/2% HPMC (manufactured by FUJIFILM Wako Pure Chemical Corporation, manufactured by Pure Chemicals)/97% distilled water (manufactured by Otsuka Pharmaceutical Factory, Inc.), and adagrasib, after being dissolved in 35% DMSO (manufactured by FUJIFILM Wako Pure Chemical Corporation)/65% Tween80 (manufactured by Sigma-Aldrich Co. LLC) to reach 100 mg/mL, was prepared with 10% Captisol (manufactured by Cyclolab Ltd.)/50 mM citrate buffer (manufactured by MUTO PURE CHEMICALS CO., LTD.). The long diameter and short diameter of tumors generated in each mouse were measured with a Digimatic Caliper (manufactured by Mitutoyo Corp.) twice a week from day 1 as the day of initiation of administration to the final day of the test.

The tumor volume was calculated by the following formula. Tumor volume (mm3) = Tumor long diameter (mm) × Tumor short diameter2 (mm2) / 2

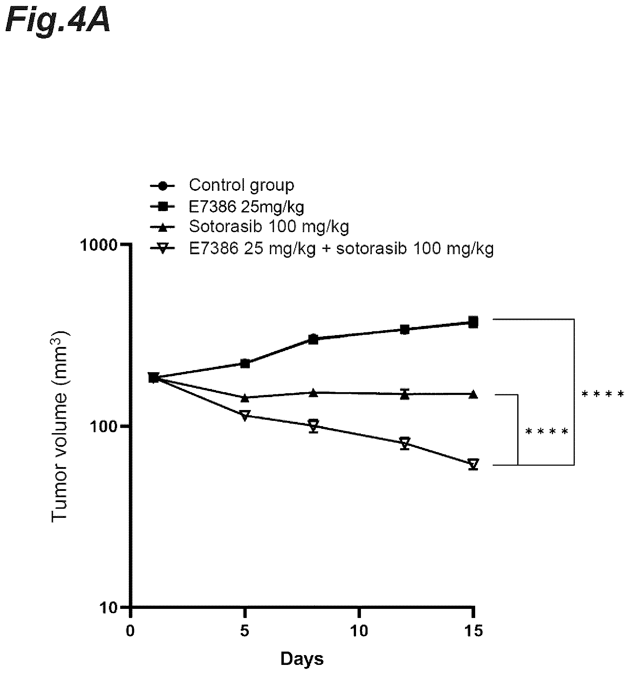

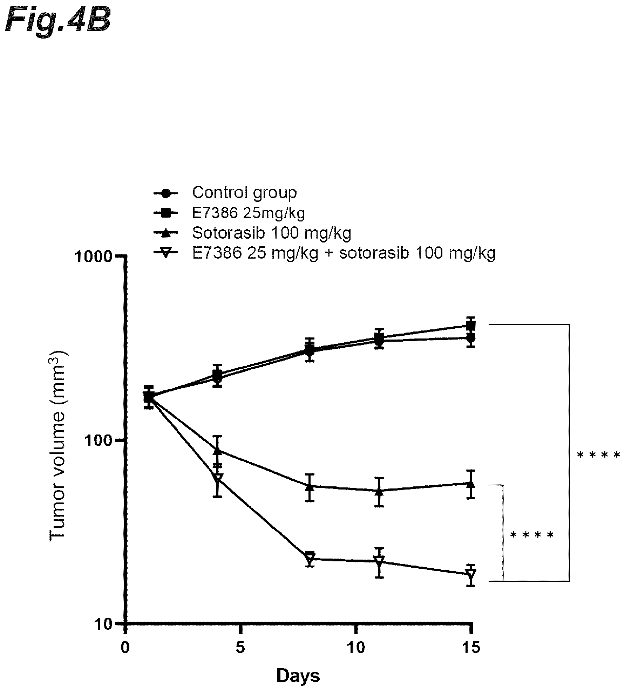

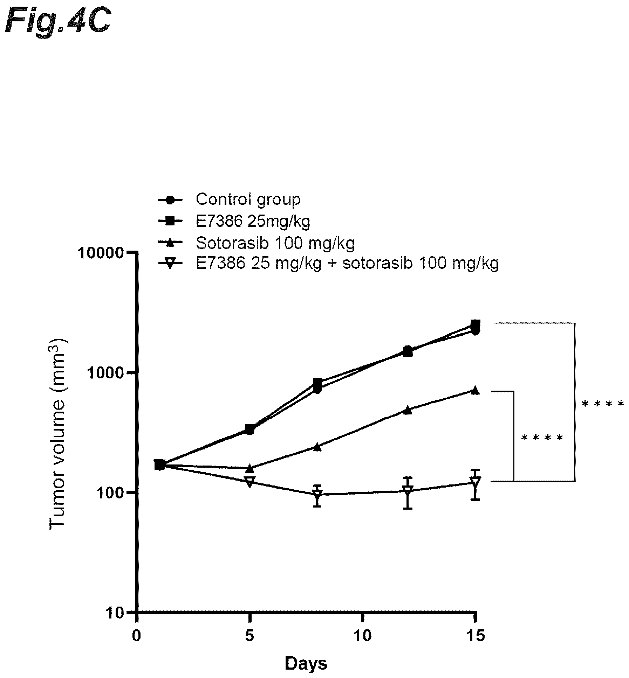

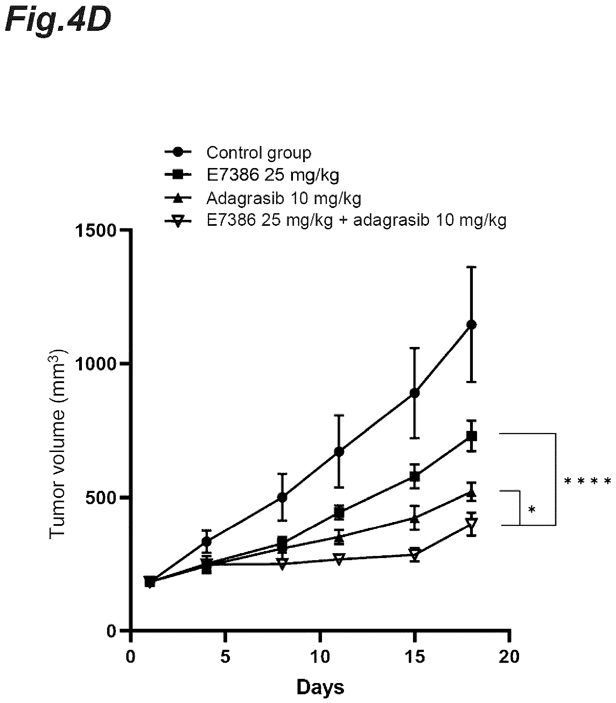

The results of measurement of tumor volume for the groups were shown as means and standard errors (SEM) in Table 6 and Figures 4A to 4D (Figure 4A: SW837, Figure 4B: MIA PaCa-2, Figure 4C: UM-UC-3, Figure 4D: NCI-H358). Numbers in Table 6 each indicate mean ± standard error (SEM) of tumor volume. In the test carried out, six mice were used per group.

The results showed that combined administration of E7386 and sotorasib exhibited significant antitumor effect compared with each drug alone in the xenograft models with the KRAS G12C mutation-positive human colorectal cancer cell line SW837, the KRAS G12C mutation-positive human pancreatic cancer cell line MIA PaCa-2, and the KRAS G12C mutation-positive human bladder cancer cell line UM-UC-3. Combined administration of E7386 and adagrasib exhibited significant antitumor effect compared with each drug alone in the xenograft model with the KRAS G12C mutation-positive human non-small cell lung cancer cell line NCI-H358. In Table 5, Table 6, and Figures 4A to 4D, E7386 indicates the compound represented by formula (I). * and **** in Figures 4A to 4D each indicate that combined administration of E7386 and sotorasib statistically significantly inhibited tumor growth compared with each drug alone (* < 0.05, ****: p < 0.0001; Repeated measures ANOVA followed by Dunnett's type multiple comparison after logarithmic transformation).

**[Table 5]**

| Cell line | Suspension medium | Transplanted cell count | Lineage of mice (breeder) | Administered drug | Day of initiation of administration | Test period |
|---|---|---|---|---|---|---|
| SW837 | Matrigel (manufactured by Corning Incorporated/HBSS (50:50) | 1 × 10⁷ cells | CAnN. Cg-Foxnlnu/CrlCrlj (Charles River) | E7386, sotorasib | Day 15 after transplantation | 15 days |
| MIA PaCa-2 | HBSS | 5 × 10⁶ cells | CAnN. Cg-Foxnlnu/CrlCrlj (Charles River) | E7386, sotorasib | Day 21 after transplantation | 15 days |
| UM-UC-3 | Matrigel (manufactured by Corning Incorporated/HBSS (50:50) | 1 × 10⁷ cells | BALB/c Slc nu/nu (Japan SLC) | E7386, sotorasib | Day 8 after transplantation | 15 days |
| NCI-H358 | Matrigel (manufactured by Corning Incorporated/HBSS (50:50) | 8 × 10⁶ cells | CAnN. Cg-Foxnlnu/CrlCrlj (Charles River) | E7386, adagrasib | Day 6 after transplantation | 18 days |

**[Table 6]**

| Cell line | Group | Day 5 | Day 8 | Day 12 | Day 15 | |
|---|---|---|---|---|---|---|
| SW837 | Control group | 223.4±3.1 | 304.8±9.0 | 338.1±10.1 | 3703±6.6 | |
| | E7386 25 mg/kg group | 221.5±9.5 | 300.9±9.0 | 340.9±17.5 | 374.4±24.2 | |
| | Sotorasib 100 mg/kg group | 143.8±3.5 | 153.7±7.0 | 150.7±8.8 | 151.0±6,6 | |
| | E7386 25 mg/kg + Sotorasib 100 mg/kg group | 114.6±4.5 | 100.5±7.9 | 80.6±5.9 | 61.8±3.8 | |

| Cell line | Group | Day 4 | Day 8 | Day 11 | Day 15 | |
|---|---|---|---|---|---|---|
| MIA PaCa-2 | Control group | 217.4±21.5 | 304.1±33.7 | 344.7±28.1 | 359.9±38.4 | |
| | E7386 25 mg/kg group | 228.5±29.2 | 312.9±43.7 | 360.0±40.9 | 419.9±43.4 | |
| | Sotorasib 100 mg/kg group | 88.2±16.9 | 56.1±9.2 | 53.0±9.3 | 58.3±10.0 | |
| | E7386 25 mg/kg + Sotorasib 100 mg/kg group | 61.6±12.3 | 22.5±1.9 | 21.9±4.0 | 18.5±2.4 | |

| Cell line | Group | Day 5 | Day 8 | Day 12 | Day 15 | |
|---|---|---|---|---|---|---|
| UM-UC-3 | Control group | 328.2±18.9 | 726.5±27.2 | 1548.6+89.4 | 2232.7±155.3 | |
| | E7386 25 mg/kg group | 337.4±20.9 | 828.5±41.9 | 1487.0±59.7 | 2526.8±123.1 | |
| | Sotorasib 100 mg/kg group | 160.3±9.5 | 240.4±17.0 | 489.1±30.7 | 715.4±51.6 | |
| | E7386 25 mg/kg + Sotorasib 100 mg/kg group | 122.6±4.1 | 95.6±18.6 | 103.0±29.2 | 121.5±33.9 | |

| Cell line | Group | Day 4 | Day 8 | Day 11 | Day 15 | Day 18 |
|---|---|---|---|---|---|---|
| NCI-H358 | Control group | 334.6±41.4 | 501.2±87.1 | 671.6+134.2 | 890.2±169.4 | 1146.7±215.3 |
| | E7386 25 mg/kg group | 251.2±8.1 | 327.4±24.9 | 445.0±26.3 | 579.1+45.1 | 729.2±56.7 |
| | Adagrasib 10 mg/kg group | 243.8±19.8 | 308.7±42.5 | 351.9±26.6 | 424.3+45.0 | 521.3±33.9 |
| | E7386 25 mg/kg + Adagrasib 10 mg/kg group | 248.7±32.1 | 250.5±16.8 | 268.1±12.0 | 285.4±25.1 | 400.5±43.2 |

## Claims

1. A pharmaceutical composition for treating a tumor, the pharmaceutical composition comprising
(6S,9aS)-N-benzyl-8-({6-[3-(4-ethylpiperazin-1-yl)azetidin-1-yl]pyri din-2-yl}methyl)-6-(2-fluoro-4-hydroxybenzyl)-4,7-dioxo-2-(prop-2-en-1-yl) hexahydro-2H-pyrazino[2,1-c][1,2,4]triazine-1(6H)-carboxamide represented by formula (I) or a pharmaceutically acceptable salt thereof, wherein
the pharmaceutical composition is administered in combination with an RAS inhibitor

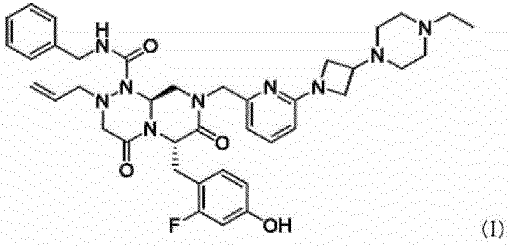

2. A pharmaceutical composition for treating a tumor, the pharmaceutical composition comprising an RAS inhibitor, wherein
the pharmaceutical composition is administered in combination with (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydr o-2H-pyrazino[2,1-c][1,2,4]triazine-1(6H)-carboxamide represented by formula (I) or a pharmaceutically acceptable salt thereof

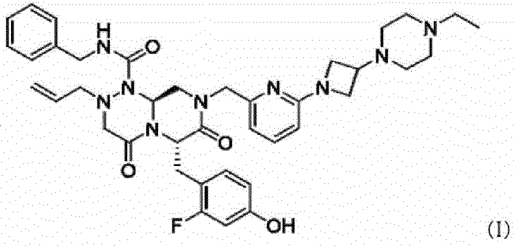

3. The pharmaceutical composition according to claim 1, wherein the (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydr o-2H-pyrazino[2,1-c][1,2,4]triazine-1(6H)-carboxamide or pharmaceutically acceptable salt thereof is administered with the RAS inhibitor simultaneously or at different times.

4. The pharmaceutical composition according to claim 2, wherein the RAS inhibitor is administered with the (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydr o-2H-pyrazino[2,1-c][1,2,4]triazine-1(6H)-carboxamide or pharmaceutically acceptable salt thereof simultaneously or at different times.

5. The pharmaceutical composition according to any one of claims 1 to 4, wherein the (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydr o-2H-pyrazino[2,1-c][1,2,4]triazine-1(6H)-carboxamide or pharmaceutically acceptable salt thereof is (6S,9aS)-N-benzyl-8-({6-[3-(4-ethylpiperazin-1-yl)azetidin-1-yl]pyridin-2-yl} methyl)-6-(2-fluoro-4-hydroxybenzyl)-4,7-dioxo-2-(prop-2-en-1-yl)hexahydr o-2H-pyrazino[2,1-c][1,2,4]triazine-1(6H)-carboxamide.

6. The pharmaceutical composition according to any one of claims 1 to 5, wherein the RAS inhibitor is a KRAS inhibitor.

7. The pharmaceutical composition according to claim 6, wherein the KRAS inhibitor is at least one selected from sotorasib, adagrasib, MRTX1133, BI-3406, BI 1701963, RG6330, LY3537982, JDQ443, RMC-6291, RMC-6236, LUNA18, ARS-1620, JNJ-74699157, GDC-6036, iExosomes, and mRNA-56713.

8. The pharmaceutical composition according to claim 6, wherein the KRAS inhibitor is sotorasib, adagrasib, or BI 1701963.

9. The pharmaceutical composition according to claim 6, wherein the KRAS inhibitor is a KRAS G12C inhibitor.

10. The pharmaceutical composition according to claim 9, wherein the KRAS G12C inhibitor is sotorasib or adagrasib.

11. The pharmaceutical composition according to any one of claims 1 to 10, wherein the tumor is at least one selected from non-small cell lung cancer, colorectal cancer, pancreatic cancer, mesothelioma, endometrial cancer, ovarian cancer, bladder cancer, bile duct cancer, gastric cancer, breast cancer, small cell lung cancer, testis cancer, small intestine cancer, appendix cancer, and neuroendocrine tumor.

12. The pharmaceutical composition according to any one of claims 1 to 10, wherein the tumor is at least one selected from non-small cell lung cancer, colorectal cancer, pancreatic cancer, endometrial cancer, and ovarian cancer.

13. The pharmaceutical composition according to any one of claims 1 to 10, wherein the tumor is at least one selected from non-small cell lung cancer and colorectal cancer.
